# FASCICULE DE BREVET EUROPEEN

(11) **EP 1 813 265 B1**
(45) Date de publication et mention de la délivrance du brevet: **20.06.2018**
(21) Numéro de dépôt: 07101220.7
(22) Date de dépôt: 26.01.2007
(51) Int. Cl.: A61K 8/81, A61K 8/34, A61Q 5/06

(54) **Composition cosmétique comprenant au moins un terpolymère, au moins un polymère fixant et au moins un polyol, procédé de traitment cosmétique des fibres kératinique et utilisation de la composition**
Kosmetische Zusammensetzung, die mindestens ein Terpolymer, mindestens ein fixierendes Polymer und mindestens ein Polyol enthält, Verfahren zur kosmetischen Behandlung von Keratinfasern und Verwendung der Zusammensetzung
Cosmetic ingredient comprising at least one terpolymer, at least one binding polymer and at least one polyhydric alcohol, method for cosmetic treatment of keratin fibres and use of the ingredient

(30) Priorité: 27.01.2006 FR 0600762; 27.01.2006 FR 0600764
(43) Date de publication de la demande: 01.08.2007
(73) Titulaire: L'OREAL, S.A., 75008 Paris (FR)
(72) Inventeur: Pasquet, Dorothée, 92270, BOIS-COLOMBES (FR); Bebot, Cécile, 92110, CLICHY (FR)
(74) Mandataire: Casalonga, Axel

(56) Documents cités:
- EP-A- 1 138 315
- EP-A- 1 502 577
- EP-A- 1 586 300
- WO-A-02/11685
- DATABASE WPI Week 199802 Derwent Publications Ltd., London, GB; AN 1998-011904 XP002404429 & JP 09 255524 A (LION CORP) 30 septembre 1997 (1997-09-30)

## Description

La présente invention concerne une composition cosmétique pour le traitement des fibres kératiniques, en particulier des fibres kératiniques humaines telles que les cheveux, comprenant au moins un terpolymère particulier, au moins un polymère fixant particulier et au moins un polyol, une utilisation de cette composition pour le soin des cheveux ainsi qu'un procédé de traitement cosmétique la mettant en oeuvre.

Dans le domaine du coiffage, en particulier parmi les produits capillaires destinés à la mise en forme et/ou au maintien de la coiffure, les compositions capillaires sont généralement constituées d'une solution le plus souvent alcoolique ou aqueuse et d'un ou de plusieurs polymères fixants en mélange avec divers adjuvants cosmétiques.

Ces compositions peuvent se présenter sous forme de gels, de lotions, de mousses ou de sprays capillaires qui sont généralement appliquées sur des cheveux mouillés avant d'effectuer un brushing ou un séchage.

En particulier, les gels capillaires sont notamment constitués d'un ou de plusieurs polymères épaississants ou agents gélifiants en association avec un ou plusieurs polymères fixants qui ont le plus souvent pour fonction de former un film à la surface des fibres kératiniques à fixer afin de réaliser des soudures entre celles-ci, structurant ou construisant ainsi la coiffure et lui apportant une tenue.

Cependant, certains épaississants utilisés habituellement avec les polymères fixants confèrent au produit une mise en forme plus ou moins facile, une tenue dans le temps limitée et une friabilité plus ou moins importante du film responsable de la fixation avec désagrégation partielle des soudures.

Il existe donc un réel besoin de trouver des compositions cosmétiques, notamment pour le coiffage, qui permettent une mise en forme aisée de la chevelure et un maintien de la coiffure durable.

De manière surprenante et avantageuse, la Demanderesse vient de découvrir qu'en associant au moins un terpolymère particulier, au moins un polymère fixant choisi parmi les homopolymères fixants non-ioniques ou les polymères fixants anioniques et au moins un polyol dans des proportions particulières, il était possible de coiffer la chevelure en lui donnant la forme souhaitée très rapidement et d'obtenir des gels conduisant à des coiffures dont le tenue dans le temps est nettement améliorée tout en obtenant une friabilité très limitée du film fixateur.

La présente invention a donc notamment pour objet une composition cosmétique pour le traitement des fibres kératiniques, en particulier des fibres kératiniques humaines telles que les cheveux, comprenant, dans un milieu cosmétiquement acceptable :
- au moins un terpolymère comportant parmi ses monomères au moins un acide carboxylique à insaturation α,β-monoéthylénique ; au moins un ester d'acide carboxylique à insaturation α,β-monoéthylénique et au moins un ester d'acide carboxylique à insaturation α,β-monoéthylénique et d'alcool gras oxyalkyléné ;
- au moins un polymère fixant choisi parmi
- les homopolymères fixants non-ioniques, le rapport entre les quantités exprimées en pourcentage en poids par rapport au poids total de la composition du terpolymère sur l'homopolymère fixant non-ionique étant supérieur ou égal à 0,2,
- au moins un polyol différent de l'homopolymère fixant non-ionique, et le rapport entre les quantités exprimées en pourcentage en poids par rapport au poids total de la composition du terpolymère sur le polyol étant inférieur à 1, et
- si la composition comprend de plus un polymère fixant anionique, alors le terpolymère se trouve en une quantité suffisante pour obtenir une viscosité supérieure à 50 cps à 25°C à un taux de cisaillement de 1s⁻¹ ;

Un autre objet de la présente invention consiste en une utilisation de la composition cosmétique selon l'invention pour la fixation des cheveux ou pour le soin des cheveux.

L'invention a également pour objet un procédé de traitement cosmétique mettant en oeuvre la composition cosmétique selon l'invention.

D'autres objets, caractéristiques, aspects et avantages de l'invention apparaîtront encore plus clairement à la lecture de la description et des exemples qui suivent.

Par le mot « composition capillaire », on entend une composition pour le maintien et/ou la fixation de la coiffure, le soin des cheveux, le maquillage des cheveux ou la coloration des cheveux.

Par le mot « coiffage », on entend le fait de fixer et/ou de maintenir la forme de la coiffure.

Au sens de la présente invention, on entend par « polymère fixant », un polymère capable de fixer et/ou de maintenir la forme de la coiffure. Les polymères fixants peuvent être utilisés sous forme solubilisée ou sous forme de dispersion de particules solides de polymères.

Selon l'invention, la composition comprend au moins un terpolymère comportant parmi ses monomères au moins un acide carboxylique à insaturation α,β-monoéthylénique ; au moins un ester d'acide carboxylique à insaturation α,β-monoéthylénique et au moins un ester d'acide carboxylique à insaturation α,β-monoéthylénique et d'alcool gras oxyalkyléné.

Préférentiellement, ces composés comprennent comme monomère au moins un acide carboxylique à insaturation α,β-monoéthylénique en C₁-C₁₀; au moins un ester d'acide carboxylique à insaturation α,β-monoéthylénique en C₃-C₁₆ et d'alcool en C₁-C₄ et au moins un ester d'acide carboxylique à insaturation α,β-monoéthylénique et d'alcool gras oxyalkyléné en C₁₀-C₂₆

De préférence, les acides carboxyliques à insaturation α,β-monoéthylénique en C₁-C₁₀ sont l'acide acrylique ou l'acide méthacrylique.

A titre d'exemple de ce type de composé, on peut citer l'ACULYN 22® vendu par la société ROHM et HAAS, qui est un terpolymère acide méthacrylique/acrylate d'éthyle/méthacrylate de stéaryle oxyalkyléné ou l'ACULYN 28® vendu par la société ROHM et HAAS, qui est un terpolymère acide méthacrylique/acrylate d'éthyle/méthacrylate de béhényle oxyalkyléné.

La masse moléculaire en poids des terpolymères utilisés dans la composition cosmétique selon la présente invention est de préférence comprise entre 500 et 20 000 000. Elle est plus particulièrement comprise entre 200 000 et 2 000 000 et encore plus préférentiellement comprise entre 400 000 et 800 000.

Le(s) terpolymère(s) selon l'invention peut ou peuvent être présents dans la composition cosmétique selon l'invention en une quantité variant de 0,05 à 30 % en poids, de préférence en une quantité variant de 0,1 à 15 % en poids et encore plus préférentiellement en une quantité variant de 0,2 à 10 % en poids, par rapport au poids total de la composition.

L'homopolymère fixant non ionique utilisable selon la présente invention est choisi parmi :
- les homopolymères de styrène ;
- les polyalkyloxazolines telles que les polyéthyloxazolines proposées par la société DOW CHEMICAL sous les dénominations PEOX 50 000, PEOX 200 000 et PEOX 500 000 ;
- les homopolymères d'acétate de vinyle tels que le produit proposé sous le nom de APPRETAN EM par la société HOECHST ou le produit proposé sous le nom de RHODOPAS A 012 par la société RHONE POULENC ou les homopolymères d'alcool de vinyle;
- les homopolymères de vinyllactame tels que la polyvinylpyrrolidone ou le polyvinylcaprolactame commercialisé sous la dénomination Luviskol® PLUS par la société BASF.
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF.

De préférence, les homopolymères non-ioniques de l'invention sont choisis parmi les homopolymères de vinyllactame.

Le poids moléculaire en nombre (PM) des polymères fixants non ioniques de l'invention est de préférence supérieur à 350.

Les groupes alkyles des homopolymères non ioniques mentionnés ci-dessus ont, de préférence, de 1 à 6 atomes de carbone.

Le ou les homopolymères fixants non-ioniques peuvent être présents dans la composition dans une quantité variant de 0,1 à 30 %, de préférence en une quantité variant de 0,2 à 20 %, et encore plus préférentiellement dans une quantité variant de 0,5 à 10 % en poids, par rapport au poids total de la composition.

La composition peut comprendre au moins un polymère fixant anionique.

Les polymères fixants anioniques sont des polymères comportant des groupements dérivés d'acide carboxylique, sulfonique ou phosphorique et ont un poids moléculaire compris entre environ 500 et 5.000.000.

Les groupements carboxyliques sont apportés par des monomères mono ou diacides carboxyliques insaturés tels que ceux répondant à la formule (I): 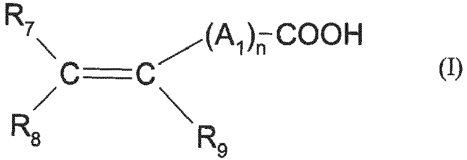 dans laquelle n est un nombre entier de 0 à 10, A1 désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R7 désigne un atome d'hydrogène, un groupement phényle ou benzyle, R8 désigne un atome d'hydrogène, un groupement alkyle inférieur ou carboxyle, R9 désigne un atome d'hydrogène, un groupement alkyle inférieur, un groupement -CH2-COOH, phényle ou benzyle.

Dans la formule précitée un radical alkyle inférieur désigne de préférence un groupement ayant 1 à 4 atomes de carbone et en particulier, méthyle et éthyle.

Les polymères fixants anioniques à groupements carboxyliques préférés selon l'invention sont :
A) Les homo- ou copolymères non réticulés d'acide acrylique ou méthacrylique ou leurs sels et en particulier les produits vendus sous les dénominations VERSICOL E ou K par la société ALLIED COLLOID et ULTRAHOLD par la société BASF. Les copolymères d'acide acrylique et d'acrylamide vendus sous la forme de leur sel de sodium sous les dénominations RETEN 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C1-C20 par exemple de lauryle tel que celui vendu par la société ISP sous la dénomination ACRYLIDONE LM et les terpolymères acide méthacrylique/ acrylate d'éthyle/ acrylate de tertiobutyle tel que le produit vendu sous la dénomination LUVIMER 100 P par la société BASF.
C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique alpha - ou beta -cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH.
D) les copolymères dérivés d'acides ou d'anhydrides carboxyliques monoinsaturés en C4-C8 choisis parmi :
   - les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées ; De tels polymères sont décrits en particulier dans les brevets US 2.047.398, 2.723.248, 2.102.113, le brevet GB 839.805 et notamment ceux vendus sous les dénominations GANTREZ AN ou ES par la société ISP.
   - les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupements acrylamide, méthacrylamide, alpha -oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.
      Ces polymères sont par exemple décrits dans les brevets français 2 350.384 et 2.357.241 de la demanderesse.
E) les polyacrylamides comportant des groupements carboxylates.

Les polymères comprenant les groupements sulfoniques sont des polymères comportant des motifs vinylsulfonique, styrène sulfonique, naphtalène sulfonique ou acrylamido alkylsulfonique. Ces polymères peuvent être notamment choisis parmi :
- les sels de l'acide polyvinylsulfonique ayant un poids moléculaire compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone
- les sels de l'acide polystyrène sulfonique les sels de sodium ayant un poids moléculaire d'environ 500.000 et d'environ 100.000 vendus respectivement sous les dénominations Flexan 500 et Flexan 130 par National Starch. Ces composés sont décrits dans le brevet FR 2.198.719.
- les sels d'acides polyacrylamide sulfoniques ceux mentionnés dans le brevet US 4.128.631 et plus particulièrement l'acide polyacrylamidoéthylpropane sulfonique vendu sous la dénomination COSMEDIA POLYMER HSP 1180 par Henkel.

Selon l'invention, les polymères fixants anioniques sont de préférence choisis parmi les copolymères d'acide acrylique tels que le terpolymère acide acrylique / acrylate d'éthyle / N-tertiobutylacrylamide vendu sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique / acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléique mono estérifié vendu sous la dénomination GANTREZ ES 425 par la société ISP, les copolymères d'acide méthacrylique et de méthacrylate de méthyle vendus sous la dénomination EUDRAGIT L par la société ROHM PHARMA, le copolymère d'acide méthacrylique et d'acrylate d'éthyle vendu sous la dénomination LUVIMER MAEX ou MAE par la société BASF, le copolymère acétate de vinyle/acide crotonique vendu sous la dénomination LUVISET CA 66 par la société BASF et le copolymère acétate de vinyle/acide crotonique greffé par du polyéthylèneglycol sous la dénomination ARISTOFLEX A par la société BASF.

Les polymères fixants anioniques les plus particulièrement préférés sont choisis parmi le copolymère méthylvinyléther / anhydride maléique mono estérifié vendu sous la dénomination GANTREZ ES 425 par la société ISP, le terpolymère acide acrylique / acrylate d'éthyle / N-tertiobutylacrylamide vendu sous la dénomination ULTRAHOLD STRONG par la société BASF, les copolymères d'acide méthacrylique et de méthacrylate de méthyle vendus sous la dénomination EUDRAGIT L par la société ROHM PHARMA, les terpolymères acétate de vinyle / tertio-butyl benzoate de vinyle / acide crotonique et les terpolymères acide crotonique / acétate de vinyle / néododécanoate de vinyle vendus sous la dénomination Résine 28-29-30 par la société NATIONAL STARCH, le copolymère d'acide méthacrylique et d'acrylate d'éthyle vendu sous la dénomination LUVIMER MAEX OU MAE par la société BASF, le terpolymère de vinylpyrrolidone / acide acrylique/méthacrylate de lauryle vendu sous la dénomination ACRYLIDONE LM par la société ISP.

Les polymères fixants anioniques de l'invention peuvent être aussi des polymères greffés à groupement siliconés, la chaîne principale pouvant être non siliconée et les greffons siliconés ou la chaîne principale pouvant être siliconée et les greffons non siliconés. A titre d'exemple de ce type de polymère, on peut citer le polymère V580 proposé par la société 3M.

Selon une variante préférée, on peut aussi utiliser à titre de polymère fixant anionique des polyuréthanes anioniques comportant ou non des motifs siliconés. A titre d'exemple de tels polyuréthanes, on peut citer les polymères LUVISET PUR et LUVISET Si PUR proposés par la société BASF.

Enfin dans une autre variante préférée de l'invention, le polymère fixant anionique est un polymère de type polyester comportant des groupements sulfoniques, on peut notamment citer comme polymère de ce type, les polymères AQ proposés par la société Eastman Chemical.

Le ou les polymères fixants anioniques peuvent être présents dans la composition dans une quantité variant de 0,1 à 30 %, de préférence en une quantité variant de 0,2 à 20 %, et encore plus préférentiellement dans une quantité variant de 0,5 à 10 % en poids, par rapport au poids total de la composition.

Les polyols utilisables dans la composition selon la présente invention sont de formule (II) : 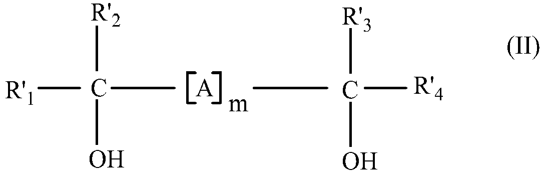 dans laquelle R'₁, R'₂, R'₃, R'₄ désignent indépendamment l'un de l'autre un atome d'hydrogène, un radical alkyle en C₁-C₆ ou un radical mono ou polyhydroxyalkyle en C₁-C₆,

A désigne un radical alkylène linéaire ou ramifié contenant de 1 à 18 atomes de carbone, ce radical comprend de 0 à 9 atomes d'oxygène, mais pas de groupement hydroxyle,
m désigne 0 ou 1.

Un premier groupe de polyols préférés est constitué des polyols de formule (II) pour laquelle m vaut 0 tels que le 1,2,3-propanetriol, le pinacol (2,3-diméthyl 2,3-butanediol), le 1,2,3-butanetriol, le 2,3-butanediol et le sorbitol.

Un deuxième groupe de polyols préférés est constitué des polyols de formule (II) pour laquelle m vaut 1 et R'₁, R'₂, R'₃, R'₄ désignent, indépendamment l'un de l'autre, un atome d'hydrogène ou un radical alkyle en C₁-C₆. Parmi ces polyols, les polyéthylèneglycols tels que par exemple le produit nommé PEG-6 ou PEG-8 dans l'ouvrage CTFA (International Cosmetic Ingrédient Dictionary, Seventh Edition) sont particulièrement préférés.

Un troisième groupe de polyols préférés est constitué des polyols de formule (II) pour laquelle m=1 et R'₁, R'₂, R'₃, R'₄ désignent, indépendamment l'un de l'autre, un atome d'hydrogène ou un radical alkyle en C₁-C₆, et dont le poids moléculaire est inférieur à 200. Parmi ces polyols, on utilise de préférence le 3-méthyl-1,3,5-pentanetriol, le 1,2,4-butanetriol, le 1,5-pentanediol, le 2-méthyl-1,3 propanediol, le 1,3-butanediol, le 3-méthyl-1,5-pentanediol, le néopentylglycol (2,2-diméthyl-1,3-propanediol), l'isoprène glycol (3-méthyl-1,3-butanediol) et l'hexylèneglycol (2-méthyl-2,4-pentanediol) et de manière encore plus préférée l'hexylèneglycol, le néopentylglycol et le 3-méthyl-1,5-pentanediol.

De préférence, le poids moléculaire (PM) du polyol est compris entre 75 et 350. Encore plus préférentiellement, le poids moléculaire est compris entre 90 et 350. Tout particulièrement, ce poids moléculaire est compris entre 95 et 350.

Le ou les polyols peut ou peuvent être présents dans la composition à une teneur comprise entre 0,1 et 30 % de préférence entre 0,5 et 20 %, et encore plus préférentiellement entre 1 et 15 % en poids du poids total de la composition cosmétique.

Selon la présente invention, ces composés se trouvent dans des proportions particulières. Le rapport entre les quantités exprimées en pourcentage en poids par rapport au poids total de la composition du terpolymère sur l'homopolymère fixant non-ionique est supérieur ou égal à 0,2, et de préférence compris entre 0,2 et 20 et le rapport entre les quantités exprimées en pourcentage en poids par rapport au poids total de la composition du terpolymère sur le polyol est inférieur à 1, et de préférence compris entre 0,05 et 1.

La composition cosmétique selon l'invention peut comprendre en outre au moins une silicone.

Dans tout ce qui suit, on entend désigner par silicone, en conformité avec l'acceptation générale, tous polymères ou oligomères organosiliciés à structure linéaire ou cyclique, ramifiée ou réticulée, de poids moléculaire variable, obtenus par polymérisation et/ou par polycondensation de silanes convenablement fonctionnalisés, et constitués pour l'essentiel par une répétition de motifs principaux dans lesquels les atomes de silicium sont reliés entre eux par des atomes d'oxygène (liaison siloxane -Si-O-Si-), des radicaux hydrocarbonés éventuellement substitués, étant directement liés par l'intermédiaire d'un atome de carbone sur lesdits atomes de silicium. Les radicaux hydrocarbonés les plus courants sont les radicaux alkyles, notamment en C₁-C₁₀, et en particulier méthyle, les radicaux fluoroalkyles dont la partie alkyle est en C₁-C₁₀, les radicaux aryles et en particulier phényle.

De préférence, la silicone est une silicone oxyalkylénée.

Par silicone oxyalkylénée, on entend toute silicone comportant au moins un groupement oxyalkyléné de type (-CₓH₂ₓO-)ₐ dans lequel x peut varier de 2 à 6, et a est supérieur ou égal à 2.

Les silicones oxyalkylénées utilisables dans la composition cosmétique sont choisies parmi les formules générales (VI), (VII), (VIII) ou (IX) suivantes: 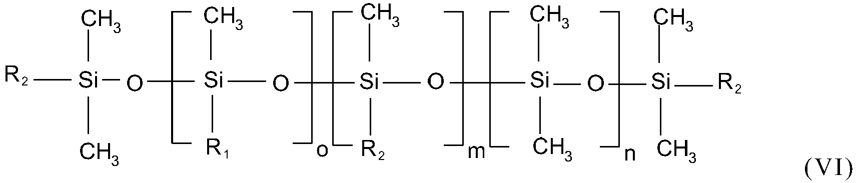 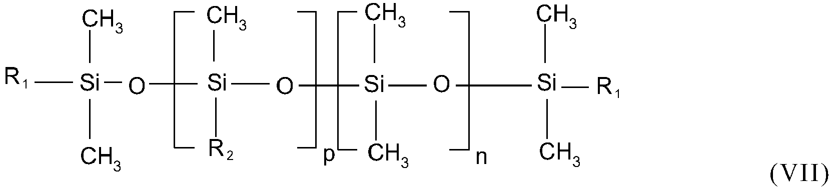 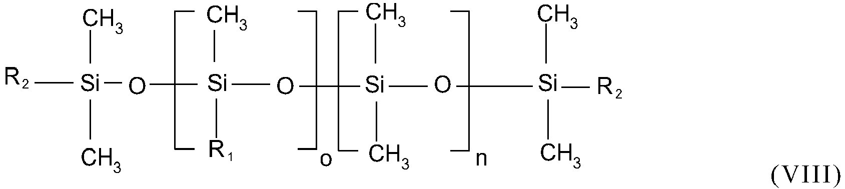 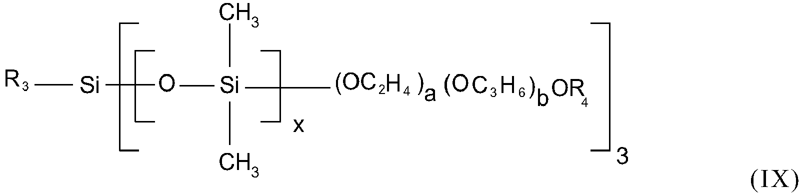 dans lesquelles:
- R₁, identique ou différent, représente un radical alkyle, linéaire ou ramifié, en C₁-C₃₀ ou phényle,
- R₂, identique ou différent, représente un radical -C_{c}H_{2c}-O-(C₂H₄O)ₐ(C₃H₆O)_{b}-R₅ ou un radical -C_{c}H_{2c}-O-(C₄H₈O)ₐ-R₅,
- R₃, R₄, identiques ou différents, désignent un radical alkyle, linéaire ou ramifié, en C₁ à C₁₂, et de préférence le radical méthyle,
- R₅, identique ou différent, est choisi parmi un atome d'hydrogène, un radical alkyle, linéaire ou ramifié, comprenant de 1 à 12 atomes de carbone, un radical alcoxy, linéaire ou ramifié, comprenant de 1 à 6 atomes de carbone, un radical acyle, linéaire ou ramifié, comprenant de 2 à 30 atomes de carbone, un radical hydroxyle, -SO₃M, aminoalcoxy en C₁-C₆ éventuellement substitué sur l'amine, aminoacyle en C₂-C₆ éventuellement substitué sur l'amine, -NHCH₂CH₂COOM, - N(CH₂CH₂COOM)₂, aminoalkyle éventuellement substitué sur l'amine et sur la chaîne alkyle, carboxyacyle en C₂-C₃₀, un groupement éventuellement substitué par un ou deux radicaux aminoalkyle substitués, -CO(CH₂)_{d}COOM, -COCHR₇(CH₂)_{d}COOM, - NHCO(CH₂)_{d}OH, -NH₃Y, un groupement phosphate,
- M, identique ou différent, désigne un atome d'hydrogène, Na, K, Li, NH₄ ou une amine organique,
- R₇ désigne un atome d'hydrogène ou un radical SO₃M,
- d varie de 1 à 10,
- m varie de 0 à 20,
- n varie de 0 à 500,
- o varie de 0 à 20,
- p varie de 1 à 50,
- a varie de 0 à 50,
- b varie de 0 à 50,
- a + b est supérieur ou égal à 2,
- c varie de 0 à 4,
- x varie de 1 à 100,
- Y représente un anion minéral ou organique monovalent tel que halogénure (chlorure, bromure), sulfate, carboxylate (acétate, lactate, citrate),
sous réserve que lorsque la silicone est de formule (VII) avec R₅ désignant hydrogène alors n est supérieur à 12.

De telles silicones sont par exemple commercialisées par la société GOLDSCHMIDT sous les dénominations commerciales ABIL WE 09, ABIL EM 90, ABIL B8852, ABIL B8851, ABIL B 8843, ABIL B8842, par la société DOW CORNING sous les dénominations FLUID DC 190, DC 3225 C, Q2-5220, Q25354, Q2-5200, par la société RHODIA CHIMIE sous les dénominations SILBIONE HUILE 70646, RHODORSIL HUILE 10634, par la société GENERAL ELECTRIC sous les dénominations SF1066, SF1188, par la société SWS SILICONES sous la dénomination SILICONE COPOLYMER F 754, par la société AMERCHOL sous la dénomination SILSOFT BEAUTY AID SL, par la société SHIN-ETSU sous la dénomination KF 351, par la société WACKER sous la dénomination BELSIL DMC 6038, par la société SILTECH sous les dénominations SILWAX WD-C, SILWAX WD-B, SILWAX WD-IS, SILWAX WSL, SILWAX DCA 100, SILTECH AMINE 65, par la société FANNING CORPORATION sous les dénominations FANCORSIL SLA, FANCORSIL LIM1, par la société PHOENIX sous la dénomination PECOSIL.

Ces silicones sont notamment décrites dans les brevets US-A-5 070 171, US-A-5149765, US-A-5093452 et US-A-5091493.

De préférence, on utilise les silicones polyoxyalkylénées répondant aux formules générales (VII) ou (VIII). Plus particulièrement, ces formules répondent à au moins une des, et de préférence toutes les, conditions suivantes :
- c est égal à 2 ou 3.
- R₁ désigne le radical méthyle.
- R₅ représente un radical méthyle, un radical acyle en C₁₂-C₂₂, CO(CH₂)_{d}COOM.
- a varie de 2 à 25 et plus particulièrement de 2 à 15.
- b est égal à 0.
- n varie de 0 à 100.
- p varie de 1 à 20.

Les silicones polyoxyalkylénées peuvent également être choisies parmi les silicones de formule (X) suivante :

([Z(R₂SiO)_{q} R'₂SiZO][(CₙH₂ₙO)ᵣ])ₛ (X)

ladite formule (X) dans laquelle:
- R₂ et R'₂, identiques ou différents, représentent un radical hydrocarboné monovalent en C₁-C₃₀,
- n est un nombre entier allant de 2 à 4,
- q est un nombre supérieur ou égal à 4, de préférence compris entre 4 et 200 et encore plus particulièrement entre 4 et 100.
- r est un nombre supérieur ou égal à 4, de préférence compris entre 4 et 200 et encore plus particulièrement entre 5 et 100.
- s est un nombre supérieur ou égal à 4, de préférence compris entre 4 et 1 000 et encore plus particulièrement entre 5 et 300.
- Z représente un groupe organique divalent qui est lié à l'atome de silicium adjacent par une liaison carbone-silicium et au bloc polyoxyalkylène (CₙH₂ₙO) par un atome d'oxygène,
- le poids moléculaire moyen de chaque bloc siloxane est compris entre environ 400 et environ 10 000, celui de chaque bloc polyoxyalkylène étant compris entre environ 300 et environ 10 000,
- les blocs siloxane représentent de 10% environ à 95% environ en poids du copolymère bloc,
- le poids moléculaire moyen en nombre du copolymère bloc pouvant aller de 2 500 à 1 000 000 et de préférence compris entre 3 000 et 200 000 et encore plus particulièrement entre 6 000 et 100 000.

R₂ et R'₂ sont préférentiellement choisis parmi le groupe comprenant les radicaux alkyles linéaires ou ramifiés comme par exemple les radicaux méthyle, éthyle, propyle, butyle, pentyle, hexyle, octyle, décyle, dodécyle, les radicaux aryles comme par exemple phényle, naphtyle, les radicaux aralkyls ou alkylaryles comme par exemple benzyle, phényléthyle, les radicaux tolyle, xylyle.

Z est de préférence -R"-, -R"-CO-, -R"-NHCO-, -R"-NH-CO-NH-R'''-, -R"-OCONH-R'''-NHCO-, où R" est un groupe alkylène divalent, linéaire ou ramifié en C₁-C₆, comme par exemple l'éthylène, le propylène ou le butylène, linéaire ou ramifié et R''' est un groupe alkylène divaient ou un groupe arylène divalent comme -C₆H₄-, -C₆H₄-C₆H₄-, -C₆H₄-CH₂-C₆H₄-, -C₆H₄-C(CH₃)₂C₆H₄-.

Encore plus préférentiellement, Z représente un radical alkylène divalent, plus particulièrement le radical -C₃H₆- ou le radical C₄H₈, linéaires ou ramifiés.

La préparation des copolymères blocs est décrite dans la demande européenne EP 0 492 657 A1, dont l'enseignement est inclus dans la présente description.

De tels produits sont par exemple commercialisés sous la dénomination SILICONE FLUID FZ-2172 par la société OSI.

Les silicones utilisées peuvent se présenter sous forme de solutions aqueuses ou éventuellement sous forme de dispersions ou d'émulsions aqueuses.

La ou les silicones utilisables dans la composition cosmétique peuvent aussi être des gommes de silicone.

Les gommes de silicone utilisables dans la composition cosmétique sont notamment des polydiorganosiloxanes ayant des poids moléculaires moyens en poids élevés compris entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylméthylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

La ou les silicones utilisables dans la composition cosmétique peuvent aussi être des silicones aminées.

Par silicone aminé, on entend toute silicone comportant au moins une fonction amine primaire, secondaire, tertiaire ou un groupement ammonium quaternaire.

Les silicones aminées utilisées dans la composition cosmétique selon la présente invention sont choisies parmi :
(a) les composés répondant à la formule (XI) suivante :

   (R¹)ₐ(T)₃₋ₐ-Si[OSi(T)₂]ₙ-[OSi(T)_{b}(R')_{2-b}]ₘ-OSi(T)₃₋ₐ-(R¹)ₐ (XI)

   dans laquelle,
   T est un atome d'hydrogène, ou un radical phényle, hydroxyle (-OH), ou alkyle en C₁-C₈, et de préférence méthyle ou alcoxy en C₁-C₈, de préférence méthoxy,
   a désigne le nombre 0 ou un nombre entier de 1 à 3, et de préférence 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R₁ est un radical monovalent de formule -CqH_{2q}L dans laquelle q est un nombre de 2 à 8 et L est un groupement aminé éventuellement quaternisé choisi parmi les groupements :
      -N(R²)-CH₂-CH₂-N(R²)₂ ;
      -N(R²)₂ ; -N⁺(R²)₃ Q- ;
      -N⁺(R²) (H)₂ Q- ;
      -N⁺(R²)₂HQ- ;
      -N(R²)-CH₂-CH₂-N⁺(R²)(H)₂ Q-,
   dans lesquels R² peut désigner un atome d'hydrogène, un phényle, un benzyle, ou un radical hydrocarboné saturé monovalent, par exemple un radical alkyle en C₁-C₂₀, et Q- représente un ion halogénure tel que par exemple fluorure, chlorure, bromure ou iodure.

En particulier, les silicones aminées correspondant à la définition de la formule (XI) sont choisies parmi les composés correspondant à la formule suivante : 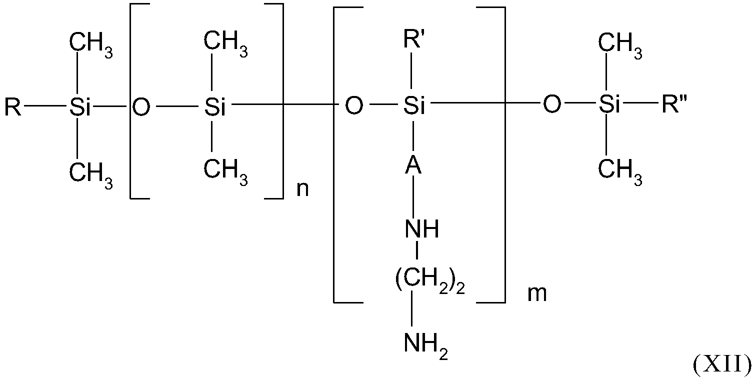 dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.

Selon une première possibilité, R, R', R", identiques ou différents, représentent un radical alkyle en C₁-C₄ ou hydroxyle, A représente un radical alkylène en C₃ et m et n sont tels que la masse moléculaire moyenne en poids du composé est comprise entre 5 000 et 500 000 environ. Les composés de ce type sont dénommés dans le dictionnaire CTFA, "amodiméthicone".

Selon une deuxième possibilité, R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000.

Dans cette catégorie de composés, on peut citer entre autres, le produit Belsil® ADM 652, commercialisée par Wacker.

Selon une troisième possibilité, R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy / alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.

Plus particulièrement, on peut citer le produit FluidWR® 1300, commercialisé par Wacker.

Selon une quatrième possibilité, R, R" représentent un radical hydroxyle, R' représente un radical méthyle et A est un radical alkylène, en C₄-C₈, de préférence en C₄. Par ailleurs, m et n sont tels que la masse moléculaire moyenne en poids du composé est comprise entre 2000 et 10⁶. Plus particulièrement, n est compris entre 0 et 1999 et m est compris entre 1 et 2000, la somme de n et m étant comprise entre 1 et 2000.

Un produit de ce type est notamment commercialisé sous la dénomination DC28299 par Dow Corning.

Notons que la masse moléculaire de ces silicones est déterminée par chromatographie par perméation de gel (température ambiante, étalon polystyrène ; colonnes µ styragem ; éluant THF ; débit de 1 mm/m ; on injecte 200 µl d'une solution à 0,5 % en poids de silicone dans le THF et l'on effectue la détection par réfractométrie et UV-métrie).

Un produit correspondant à la définition de la formule (XI) est en particulier le polymère dénommé dans le dictionnaire CTFA "triméthylsilylamodiméthicone", répondant à la formule (XIII) suivante: 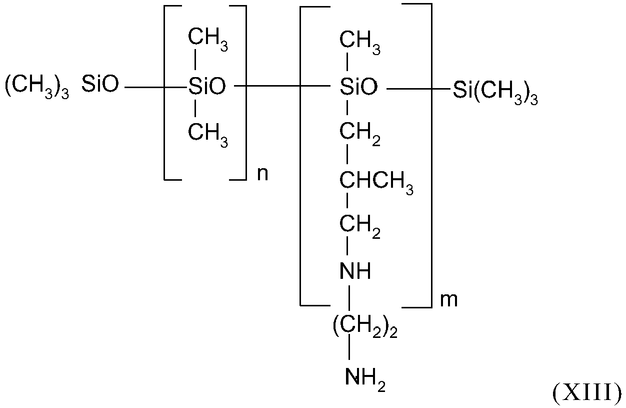 dans laquelle n et m ont les significations données ci-dessus conformément à la formule (XI).

De tels composés sont décrits par exemple dans EP 95238; un composé de formule (XIII) est par exemple vendu sous la dénomination Q2-8220 par la société OSI.
(b) les composés répondant à la formule (XIV) suivante : 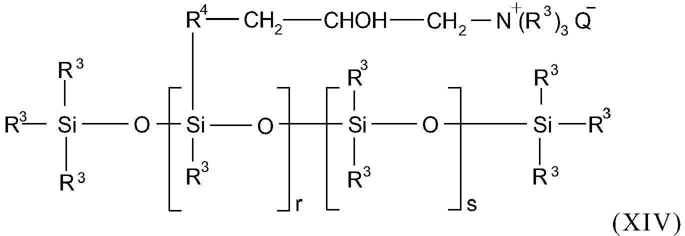 dans laquelle,
   R³ représente un radical hydrocarboné monovalent en C₁-C₁₈, et en particulier un radical alkyle en C₁-C₁₈, ou alcényle en C₂-C₁₈, par exemple méthyle ;
   R⁴ représente un radical hydrocarboné divalent, notamment un radical alkylène en C₁-C₁₈ ou un radical alkylèneoxy divalent en C₁-C₁₈, par exemple en C₁-C₈;
   Q⁻ est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.
      De tels composés sont décrits plus particulièrement dans le brevet US 4185087.
      Un composé entrant dans cette classe est celui vendu par la Société Union Carbide sous la dénomination "Ucar Silicone ALE 56".
c) les silicones ammonium quaternaire de formule (XV) : 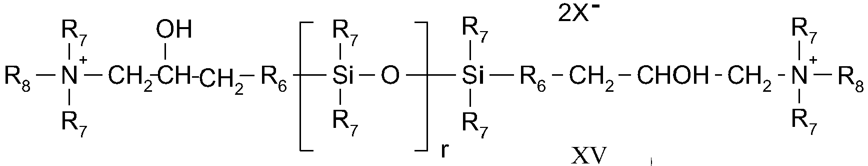 dans laquelle :
   R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇ ;
   X- est un anion tel qu'un ion halogénure, notamment chlorure ou un sel d'acide organique (acétate ...);
   r représente une valeur statistique moyenne de 2 à 200 et en particulier de 5 à 100 ;
      Ces silicones sont par exemple décrites dans la demande EP-A-0530974.
d) les silicones aminées de formule (XVI) : 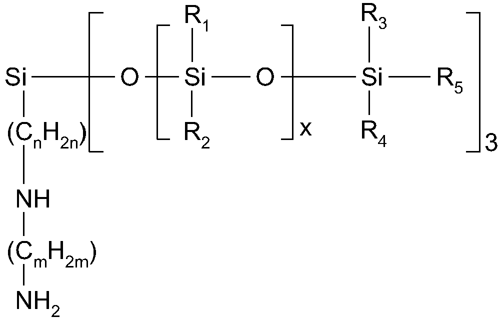 dans laquelle :
   - R₁, R₂, R₃ et R₄, identiques ou différents, désignent un radical alkyle en C₁-C₄ ou un groupement phényle,
   - R₅ désigne un radical alkyle en C₁-C₄ ou un groupement hydroxyle,
   - n est un entier variant de 1 à 5,
   - m est un entier variant de 1 à 5,
   et dans laquelle x est choisi de manière telle que l'indice d'amine soit compris entre 0,01 et 1 meq/g.

Les silicones particulièrement préférées sont les polysiloxanes à groupements aminés tels que les amodiméthicones ou les triméthylsilylamodiméthicones (CTFA 4ème édition 1997), et encore plus particulièrement les silicones à groupements ammonium quaternaire.

Lorsque ces composés sont mis en oeuvre, une forme de réalisation particulièrement intéressante est leur utilisation conjointe avec des agents de surface cationiques et/ou non ioniques.

A titre exemple, on peut utiliser le produit vendu sous la dénomination "Emulsion Cationique DC 929" par la Société Dow Corning, qui comprend, outre l'amodiméthicone, un agent de surface cationique comprenant un mélange de produits répondant à la formule : 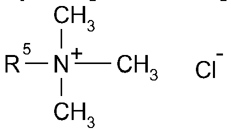 dans laquelle, R⁵ désigne des radicaux alcényle et/ou alcoyle en C₁₄-C₂₂ dérivés des acides gras du suif, et connu sous la dénomination CTFA "tallowtrimonium chloride",en association avec un agent de surface non ionique de formule :
C₉H₁₉-C₆H₄-(OC₂H₄)₁₀-OH, connu sous la dénomination CTFA "Nonoxynol 10".

On peut également utiliser par exemple le produit vendu sous la dénomination "Emulsion Cationique DC 939" par la Société Dow Corning, qui comprend, outre l'amodiméthicone, un agent de surface cationique qui est le chlorure de triméthylcétylammonium et un agent de surface non ionique de formule : C₁₃H₂₇-(OC₂H₄)₁₂-OH, connu sous la dénomination CTFA "tridéceth-12".

Un autre produit commercial utilisable selon l'invention est le produit vendu sous la dénomination "Dow Corning Q2 7224" par la Société Dow Corning, comportant en association le triméthylsilylamodiméthicone de formule (C) décrite ci-dessus, un agent de surface non ionique de formule : C₈H₁₇-C₆H₄-(OCH₂CH₂)₄₀-OH, connu sous la dénomination CTFA "octoxynol-40", un second agent de surface non ionique de formule: C₁₂H₂₅-(OCH₂-CH₂)₆-OH, connu sous la dénomination CTFA "isolaureth-6", et du propylèneglycol.

La composition cosmétique selon l'invention peut comprendre en outre un alcool gras.

Par alcool gras, on entend au sens de la présente invention, tout alcool gras pur saturé ou insaturé, linéaire ou ramifié comportant au moins 8 atomes de carbone. L'alcool gras peut être oxyalkyléné ou glycérolé.

L'alcool gras peut présenter la structure R-OH, dans laquelle R désigne un radical saturé ou insaturé, linéaire ou ramifié, comportant de 8 à 40 atomes de carbone et de préférence de 8 à 30 ; R désigne de préférence un groupement alkyle en C₁₂-C₂₄ ou alkényle en C₁₂-C₂₄. R peut être substitué par un ou plusieurs groupements hydroxy.

A titre d'exemple d'alcools gras, on peut citer les alcools laurique, cétylique, stéarylique, oléïque, béhénique, linoléique, undécylénique, palmitoléïque, arachidonique, érucique et leurs mélanges.

L'alcool gras peut représenter un mélange d'alcools gras, ce qui signifie que dans un produit commercial peuvent coexister plusieurs espèces d'alcools gras, sous forme d'un mélange.

A titre de mélange d'alcools gras, on peut citer l'alcool cétylstéarylique ou cétéarylique.

Avantageusement, l'alcool gras non oxyalkyléné est solide ou pâteux à la température de 25°C. Par « alcool gras solide ou pâteux à 25°C », on entend au sens de la présente invention un alcool gras présentant une viscosité mesurée avec un rhéomètre avec un taux de cisaillement de 1s⁻¹ supérieure ou égale à 1 Pa.s.

De préférence, les alcools gras utilisés dans la composition cosmétique selon l'invention sont l'alcool cétylique et l'alcool cétéarylique.

Le ou les alcools gras sont présents dans la composition dans une quantité variant de 0,1 à 30 %, de préférence en une quantité variant de 0,2 à 20 %, et encore plus préférentiellement dans une quantité variant de 0,5 à 10 % en poids, par rapport au poids total de la composition.

La composition cosmétique peut comprendre en outre un ou plusieurs polymères fixants additionnels différents des polymères de l'invention.

Les polymères fixants utilisables dans la composition cosmétique selon l'invention sont notamment choisis parmi les polymères cationiques, amphotères ou non ioniques et leurs mélanges autres que les homopolymères fixants non-ioniques décrits ci-dessus.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien B et C font partie d'une chaîne d'un polymère à motif éthylène alpha , beta -dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alphachloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain US 3 836 537.
2) les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
      Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertiooctyl acrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
      Les comonomères basiques préférés sont des méthacrylates d'aminoéthyle, de butyl aminoéthyle, de N,N'-diméthylaminoéthyle, de N-tertio-butylaminoéthyle.
      On utilise particulièrement les copolymères dont la dénomination CTFA (4ème Ed., 1991) est Octylacrylamide/acrylates /butylaminoethy lmethacrylate copolymer tels que les produits vendus sous la dénomination AMPHOMER ou LOVOCRYL 47 par la société NATIONAL STARCH.
(3) les polyamino amides réticulés et alcoylés partiellement ou totalement dérivant de polyaminoamides de formule générale (XVIII):

   ⁅CO-R₁₀-co-z⁆ (XVIII)

   dans laquelle R10 représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical (XIX) 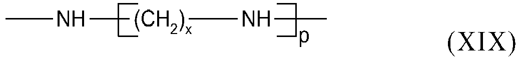 où x=2 et p=2 ou 3, ou bien x=3 et p=2 ce radical dérivant de la diéthylène triamine, de la triéthylène tétraamine ou de la dipropylène triamine;
   b) dans les proportions de 0 à 40 moles % le radical. (XX) ci-dessus, dans lequel x=2 et p=1 et qui dérive de l'éthylènediamine, ou le radical dérivant de la pipérazine : 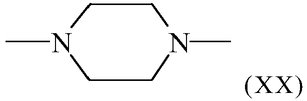
   c) dans les proportions de 0 à 20 moles % le radical -NH-(CH2)6-NH-dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
      Les alcanes sultones utilisées dans l'alcoylation sont de préférence la propane ou la butane sultone, les sels des agents d'alcoylation sont de préférence les sels de sodium ou de potassium.
(4) les polymères comportant des motifs zwittérioniques de formule (XXI): 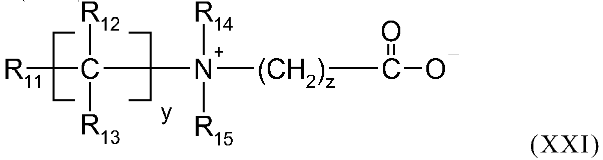 dans laquelle R11 désigne un groupement insaturé polymérisable tel qu'un groupement acrylate, méthacrylate, acrylamide ou méthacrylamide, y et z représente un nombre entier de 1 à 3, R12 et R13 représentent un atome d'hydrogène, méthyle, éthyle ou propyle, R14 et R15 représentent un atome d'hydrogène ou un radical alkyle de telle façon que la somme des atomes de carbone dans R14 et R15 ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de méthyle / diméthyl carboxyméthylammonio éthylméthacrylate de méthyle tel que le produit vendu sous la dénomination DIAFORMER Z301 par la société SANDOZ.
(5) les polymères dérivés du chitosane comportant des motifs monomères répondant aux formules suivantes : 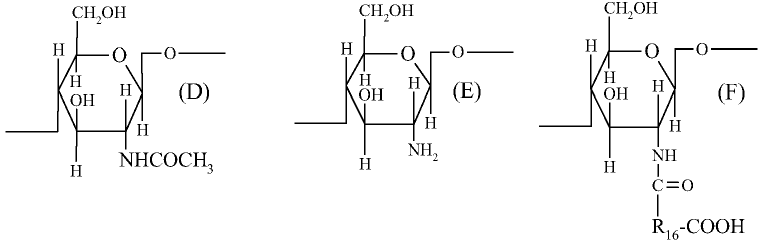 le motif D étant présent dans des proportions comprises entre 0 et 30%, le motif E dans des proportions comprises entre 5 et 50% et le motif F dans des proportions comprises entre 30 et 90%, étant entendu que dans ce motif F, R16 représente un radical de formule : 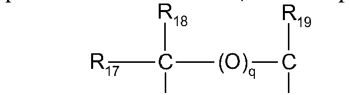 dans laquelle si q=0, R17, R18 et R19, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcolylthio, sulfonique, un reste alcoylthio dont le groupe alcoyle porte un reste amino, l'un au moins des radicaux R17, R18 et R19 étant dans ce cas un atome d'hydrogène ;
   ou si q=1, R17, R18 et R19 représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères comportant un ou plusieurs motifs répondant à la formule générale (XXII) par exemple décrits dans le brevet français 1 400 366 : 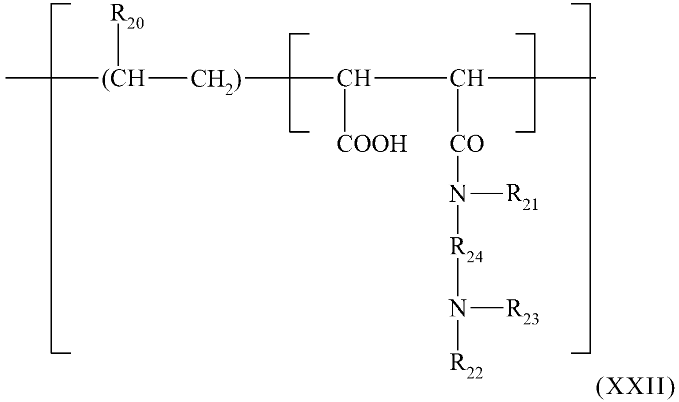 dans laquelle R20 représente un atome d'hydrogène, un radical CH3O, CH3CH2O, phényle, R21 désigne l'hydrogène ou un radical alkyle inférieur tel que méthyle, éthyle, R22 désigne l'hydrogène ou un radical alkyle inférieur tel que méthyle, éthyle, R23 désigne un radical alkyle inférieur tel que méthyle, éthyle ou un radical répondant à la formule : -R24-N(R22)2, R24 représentant un groupement -CH2-CH2-, - CH2-CH2-CH2- , -CH2-CH(CH3)- , R22 ayant les significations mentionnées ci-dessus,
   ainsi que les homologues supérieurs de ces radicaux et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule (XXIII):

      -D-X-D-X-D-

      où D désigne un radical 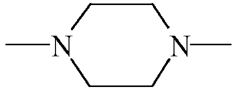 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne.
   b) Les polymères de formule (XXIV): -

      D-X-D-X-

      -où D désigne un radical 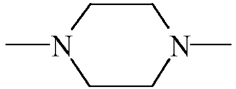 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C1-C5)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères fixants amphotères particulièrement préférés selon l'invention sont ceux de la famille (3) tels que les copolymères dont la dénomination CTFA est Octylacrylamide/acrylates/ butylaminoethylmethacrylate copolymer tels que les produits vendus sous les dénominations AMPHOMER, AMHOMER LV 71 ou LOVOCRYL 47 par la société NATIONAL STARCH et ceux de la famille (4) tels que les copolymère de méthacrylate de méthyle / diméthyl carboxyméthylammonio éthylméthacrylate de méthyle par exemple vendu sous la dénomination DIAFORMER Z301 par la société SANDOZ.

Les polymères fixants non ioniques utilisables selon la présente invention sont choisis par exemple parmi :
- les copolymères de vinylpyrrolidone et d'acétate de vinyle ;
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST;
- les copolymères d'acétate de vinyle et d'ester maléïque par exemple de maléate de dibutyle tels que le produit proposé sous le nom de APPRETAN MB EXTRA par la société HOECHST ;
- les copolymères d'esters acryliques tels que par exemple les copolymères d'acrylates d'alkyle et de méthacrylates d'alkyles tels que les produits proposés par la société ROHM&HAAS sous les dénominations PRIMAL AC-261 K et EUDRAGIT NE 30 D, par la société BASF sous les dénominations ACRONAL 601, LUHYDRAN LR 8833 ou 8845, par la société HOECHST sous les dénominations APPRETAN N 9213 ou N9212;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la société NIPPON ZEON ou ceux proposés sous la dénomination CJ 0601 B par la société ROHM & HAAS ; les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS ;
- les copolymères d'acrylate d'alkyle et d'uréthanne tels que le produit 8538-33 par la société NATIONAL STARCH ;
- les gommes de guar non ioniques chimiquement modifiées ou non modifiées.

Les gommes de guar non ioniques non modifiées sont par exemple les produits vendus sous la dénomination VIDOGUM GH 175 par la société UNIPECTINE et sous la dénomination JAGUAR C par la société MEYHALL.

Les gommes de guar non-ioniques modifiées utilisables selon l'invention sont de préférence modifiées par des groupements hydroxyalkyle en C1-C6. On peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société MEYHALL, ou sous la dénomination GALACTASOL 4H4FD2 par la société AQUALON.

Les radicaux alkyle des polymères non ioniques ont de 1 à 6 atomes de carbone sauf mention contraire.

Les polymère non ionique qui conviennent tout particulièrement pour la réalisation des compositions conformes à l'invention sont ceux choisis parmi:
- les copolymères de vinyllactame tels que les copolymères de vinylpyrrolidone et d'acétate de vinyle et les copolymères vinylpyrrolidone/ acétate de vinyle/ propionate de vinyle
- les copolymères d'acétate de vinyle et d'ester acrylique tels que le RHODOPAS AD 310 (RHONE POULENC)
- les copolymères d'acétate de vinyle et d'éthylène tels que APPRETAN TV (HOECHST)
- les copolymères d'acétate de vinyle et d'ester maléique tels que APPRETAN MB EXTRA (HOECHST)
- les copolymères d'esters acryliques tels que PRIMAL AC-261 K (ROHM & HAAS), ACRONAL 601 (BASF) ou APPRETAN N 9213 (HOECHST)
- les copolymères d'acrylonitrile et d'un monomère non-ionique tels que CJ 0601 B (ROHM & HAAS)
- les copolymères d'acrylate d'alkyle et d'uréthane tels que 8538-33 (NATIONAL STARCH).

Selon l'invention, on peut également utiliser les polymères fixants de type siliconés greffés comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule :

   CH₂=C(CH₃)-COO-(CH₂)₃-Si(CH₃)₂-O-[Si(CH₃)₂]ᵥ-Si(CH₃)₂-(CH₂)₃-CH₃

   avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut aussi utiliser, comme polymères fixants, des polyuréthannes fonctionnalisés ou non, siliconés ou non.

Selon l'invention, le ou les polymères fixants additionnels peuvent représenter de 0,1 % à 10 % en poids, de préférence de 0,5 % à 5% en poids par rapport au poids total de la composition finale.

La composition cosmétique selon l'invention peut en outre comprendre un ou plusieurs "agents d'ajustement de la rhéologie" autres que les polymères de l'invention.

Les agents d'ajustement de la rhéologie peuvent être choisis parmi les amides d'acides gras (diéthanol- ou monoéthanol-amide de coprah, monoéthanolamide d'acide alkyl éther carboxylique oxyéthyléné). Ces agents d'ajustement de la rhéologie peuvent être aussi des polymères épaississants ioniques ou non, associatifs ou non.

En ce qui concerne les polymères épaississants non associatifs, il est tout d'abord rappelé qu'au sens de la présente invention, les polymères épaississants non associatifs sont des polymères épaississants ne contenant pas de chaîne grasse en C10-C30.

Parmi les polymères épaississants non associatifs présents, on peut citer les homopolymères d'acide acrylique réticulés, homopolymères réticulés d'acide 2-acrylamido-2-méthyl-propane sulfonique et leurs copolymères réticulés d'acrylamide, les homopolymères d'acrylate d'ammonium ou les copolymères d'acrylate d'ammonium et d'acrylamide, les gommes de guar non ioniques, les gommes de biopolysaccharides d'origine microbienne, les gommes issues d'exudats végétaux, les dérivés de celluloses ; les pectines et les alginates, seuls ou en mélanges.

Une première famille de polymères épaississants non associatifs convenable est représentée par les homopolymères d'acide acrylique réticulés.

Parmi les homopolymères de ce type, on peut citer ceux réticulés par un éther allylique d'alcool de la série du sucre, comme par exemple les produits vendus sous les noms CARBOPOLS 980, 981, 954, 2984 et 5984 par la société NOVEON ou les produits vendus sous les noms SYNTHALEN M et SYNTHALEN K par la société 3 VSA.

Les polymères épaississants non associatifs peuvent aussi être choisis parmi les homopolymères réticulés d'acide 2-acrylamido-2-méthylpropane sulfonique et leurs copolymères réticulés d'acrylamide.

En ce qui concerne ces homopolymères et copolymères, qui peuvent être partiellement ou totalement neutralisés, on peut citer les polymères comprenant de 90 à 99,9% en poids, par rapport au poids total du polymère, de motifs de formule (j) suivante : 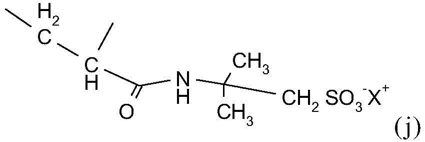 dans laquelle X+ désigne un cation ou un mélange de cations, ou un proton.

Plus particulièrement les cations sont choisis parmi les métaux alcalins (comme le sodium, le potassium), les ions ammonium substitués ou non par un à trois radicaux alkyle, identiques ou différents, comprenant 1 à 6 atomes de carbone, éventuellement porteur d'au moins un radical hydroxyle, les cations dérivant de la N-méthyl-glucamine, d'acides aminés basiques comme l'arginine et la lysine. De préférence, le cation est un ion ammonium ou sodium.

Par ailleurs, le polymère comprend de 0,01 à 10% en poids, par rapport au poids total du polymère, de motifs réticulants provenant d'au moins un monomère ayant au moins deux insaturation éthyléniques (double liaison carbone-carbone).

Les monomères de réticulation ayant au moins deux insaturations éthyléniques sont choisis par exemple parmi l'éther diallylique, le triallylcyanurate, le diallylmaléate, le (méth)acrylate d'allyle, le dipropylèneglycol-diallyléther, les polyglycol-diallyléthers, le triéthylèneglycol-divinyléther, l'hydroquinone-diallyl-éther, le tétrallyl-oxéthanoyle, le di(méth)acrylate de tétra- ou di-éthylèneglycol, la triallylamine, la tétraallyléthylènediamine le triméthylolpropane-diallyléther, le triméthylolpropane triacrylate, le méthylène-bis(méth)acrylamide ou le divinylbenzène, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Pour plus de détails au sujet de ces polymères, on pourra se reporter au document EP 815828.

Parmi les copolymères réticulés d'acide 2-acrylamido-2-méthyl-propane sulfonique et d'acrylamide partiellement ou totalement neutralisés, on peut citer en particulier le produit décrit dans l'exemple 1 du document EP 503 853 et l'on pourra se reporter à ce document pour ce qui a trait à ces polymères.

La composition peut de même comprendre, à titre de polymères épaississants non associatifs, les homopolymères d'acrylate d'ammonium ou les copolymères d'acrylate d'ammonium et d'acrylamide.

A titre d'exemples d'homopolymères d'acrylate d'ammonium, on peut citer le produit vendu sous le nom MICROSAP PAS 5193 par la société HOECHST. Parmi les copolymères d'acrylate d'ammonium et d'acrylamide, on peut citer le produit vendu sous le nom BOZEPOL C NOUVEAU ou le produit PAS 5193 vendus par la société HOECHST. On pourra notamment se référer aux documents FR 2 416 723, US 2798053 et US 2923692 pour ce qui a trait à la description et à la préparation de tels composés.

La composition peut aussi comprendre des homopolymères de diméthylaminoéthyl-méthacrylate quaternisé par le chlorure de méthyle ou les copolymères de diméthylamino-éthylméthacrylate quaternisé par le chlorure de méthyle et d'acrylamide.

Parmi les homopolymères de ce type, on peut citer les produits vendus sous les noms SALCARE 95 et SALCARE 96 par la société CIBA-ALLIED COLLOIDS. Parmi les copolymères de cette famille, on peut citer le produit SALCARE SC92 vendu par CIBA-ALLIED COLLOIDS ou le produit PAS 5194 vendu par HOECHST. Ces polymères sont notamment décrits et préparés dans le document EP 395282 auquel on pourra se référer.

La composition peut aussi comprendre des gommes de guar non ioniques, comme par exemple les gommes de guar non ioniques non modifiées vendues sous la dénomination VIDOGUM GH 175 par la société UNIPECTINE et sous la dénomination JAGUAR C par la société MEYHALL.

Les gommes de guar non ioniques utilisables selon l'invention sont de préférence modifiées par des groupements hydroxyalkyle en C1-C6. Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants, tels que par exemple des oxydes de propylène, avec la gomme de guar, de façon à obtenir une gomme de guar modifiée par des groupements hydroxypropyle.

Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2 et.

De telles gommes de guar non ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société MEYHALL ou sous la dénomination GALACTASOL 4H4FD2 par la société AQUALON.

A titre de polymères épaississants non associatifs convenables, on peut aussi mentionner les gommes de biopolysaccharides d'origine microbienne telles que les gommes de scléroglucane ou de xanthane.

Conviennent aussi les gommes issues d'exudats végétaux, telles que les gommes arabiques, gommes Ghatti, gommes Karaya et Tragacanthe ; les hydroxypropyl- ou carboxyméthyl- celluloses ; les pectines et les alginates.

Ces polymères sont bien connus de l'homme de l'art et sont notamment décrits dans l'ouvrage de Robert L. DAVIDSON intitulé "Handbook of Water soluble gums and resins" édité chez Mc Graw Hill Book Company (1980).

Parmi les agents épaississants, on préfère plus particulièrement utiliser les systèmes épaississants à base de polymères associatifs bien connus de l'homme de l'art et notamment de nature non ionique, anionique, cationique ou amphotère.

Il est rappelé que les polymères associatifs sont des polymères hydrophiles capables, dans un milieu aqueux, de s'associer réversiblement entre eux ou avec d'autres molécules. Leur structure chimique comprend plus particulièrement au moins une zone hydrophile et au moins une zone hydrophobe. Par groupement hydrophobe, on entend un radical ou polymère à chaîne hydrocarbonée, saturée ou non, linéaire ou ramifiée, comprenant au moins 8 atomes de carbone, de préférence de 10 à 30 atomes de carbone, en particulier de 12 à 30 atomes de carbone et plus préférentiellement de 18 à 30 atomes de carbone. Préférentiellement, le groupement hydrocarboné provient d'un composé monofonctionnel. A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

La composition peut donc comprendre au moins un polymère associatif, autre que les polymères de l'invention, choisi parmi les polyuréthanes associatifs, plus particulièrement cationiques ou non ioniques, les dérivés de cellulose associatifs, plus particulièrement cationiques ou non ioniques, les vinyllactames associatifs, les polyacides insaturés associatifs, les aminoplaste-éthers associatifs, les polymères ou copolymères associatifs comprenant au moins un monomère à insaturation éthylénique à groupement sulfonique, seuls ou en mélanges.

Parmi les polymères épaississants associatifs, on peut citer les dérivés de polyuréthanes associatifs, comme ceux obtenus par polymérisation :
- environ 20% à 70% en poids d'un acide carboxylique à insaturation α,β-monoéthylénique,
- environ 20 à 80% en poids d'un monomère à insaturation α,β-monoéthylénique non-tensioactif différent du précédent,
- environ 0,5 à 60% en poids d'un mono-uréthane non-ionique qui est le produit de réaction d'un tensioactif monohydroxylé avec un monoisocyanate à insaturation monoéthylénique.

De tels sont notamment décrits dans EP 173109 et plus particulièrement dans l'exemple 3. Plus précisément, ce polymère est un terpolymère acide méthacrylique / acrylate de méthyle / diméthyl métaisopropényl benzyl isocyanate d'alcool béhényle éthoxylé (40OE) en dispersion aqueuse à 25%.Ce produit est proposé sous la référence VISCOPHOBE DB1000 par la Société AMERCHOL.

Conviennent aussi les polyuréthanes associatifs cationiques dont la famille a été décrite dans la demande FR 0009609. Elle peut être représentée plus particulièrement par la formule générale (A) suivante :

R-X-(P)n-[L-(Y)m]r-L'-(P')p-X'-R' (A)

dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25 ;
n, m, et p valent chacun indépendamment des autres entre 0 et 1000 ;
la molécule contenant au moins une fonction amine protonée ou quaternisée et au moins un groupement hydrophobe.

Dans un mode de réalisation très avantageux, les seuls groupements hydrophobes de ces polyuréthanes sont les groupes R et R' situés aux extrémités de chaîne.

Selon un premier mode de réalisation préféré, le polyuréthane associatif correspond à la formule (A) dans laquelle R et R' représentent tous les deux indépendamment un groupement hydrophobe ; X, X' représentent chacun un groupe L" ; n et p valent entre 1 et 1000 et L, L', L", P, P', Y et m ont la signification indiquée que dans la formule (A).

Selon un autre mode de réalisation préféré de l'invention, le polyuréthane associatif correspond à la formule (A) dans laquelle R et R' représentent tous les deux indépendamment un groupement hydrophobe, X, X' représentent chacun un groupe L", n et p valent 0, et L, L', L", Y et m ont la signification que dans la formule (A) indiquée auparavant.

Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Conformément à un autre mode de réalisation préféré de l'invention, le polyuréthane associatif correspond à la formule (A) dans laquelle R et R' représentent tous les deux indépendamment un groupement hydrophobe ; X et X' représentent tous les deux indépendamment un groupement comportant une amine quaternaire ; n et p valent zéro, et L, L', Y et m ont la signification indiquée dans la formule (A).

La masse moléculaire moyenne en nombre des polyuréthanes associatifs cationiques est habituellement comprise entre 400 et 500000, en particulier entre 1000 et 400000 et idéalement entre 1000 et 300000 g/mol.

Lorsque X et/ou X' désignent un groupement comportant une amine tertiaire ou quaternaire, X et/ou X' peuvent représenter l'une des formules suivantes : 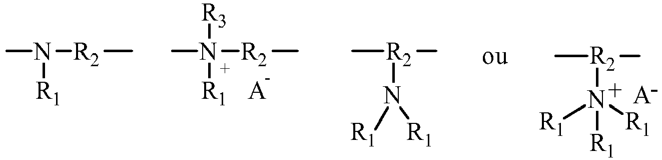 pour X 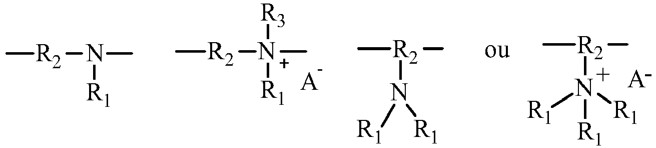 pour X' dans lesquelles :
R2 représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R1 et R3, identiques ou différents, désignent un radical alkyle ou alcényle en C1-C30, linéaire ou ramifié, un radical aryle, l'un au moins des atomes de carbone pouvant être remplacé par un hétéroatome choisi parmi N, S, O, P ;
A- est un contre-ion physiologiquement acceptable.

Les groupements L, L' et L" représentent un groupe de formule : 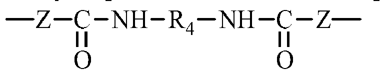 dans laquelle :
Z représente -O-, -S- ou -NH- ; et
R4 représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 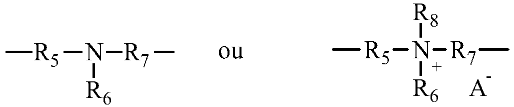 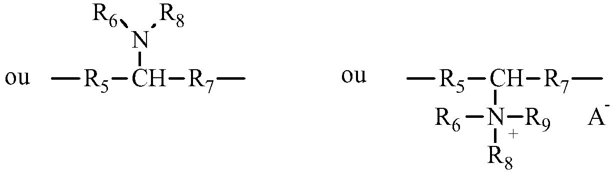 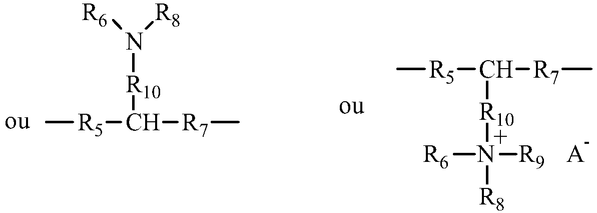 dans lesquelles :
R5 et R7 ont les mêmes significations que R2 défini précédemment ;
R6, R8 et R9 ont les mêmes significations que R1 et R3 définis précédemment ;
R10 représente un groupe alkylène, linéaire ou ramifié, éventuellement insaturé et pouvant contenir un ou plusieurs hétéroatomes choisis parmi N, O, S et P ;
A- est un contre-ion cosmétiquement acceptable.

En ce qui concerne la signification de Y, on entend par groupement hydrophile, un groupement hydrosoluble polymérique ou non. A titre d'exemple, on peut citer, lorsqu'il ne s'agit pas de polymères, l'éthylèneglycol, le diéthylèneglycol et le propylèneglycol. Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs de formule (A) sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" de la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (A) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.

Comme indiqué précédemment, ce composé peut comporter plus d'un motif à fonction amine. Il s'agit alors d'un polymère portant une répétition du motif à fonction amine.

Ce type de composés peut être représenté par l'une des formules suivantes :

HZ-(P)ₙ-ZH, ou HZ-(P')ₚ-ZH

dans lesquelles Z, P, P', n et p sont tels que définis plus haut.

A titre d'exemples de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (A) est un diisocyanate correspondant à la formule O=C=N-R₄-N=C=O, dans laquelle R₄ est défini plus haut.

On peut citer notamment le méthylènediphényl-diisocyanate, le méthylène cyclohexane diisocyanate, l'isophorone-diisocyanate, le toluènediisocyanate, le naphtalène diisocyanate, le butanediisocyanate, l'hexane diisocyanate.

Un troisième composé entrant dans la préparation du polyuréthane de formule (A) est un composé hydrophobe destiné à former les groupes hydrophobes terminaux du polymère de formule (A).

Ce composé est constitué d'un groupe hydrophobe et d'une fonction à hydrogène labile, par exemple une fonction hydroxyle, amine primaire ou secondaire, ou thiol.

A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné α-hydroxyle.

Le groupe hydrophobe du polyuréthane de formule (A) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.

A titre d'exemples, on peut citer, lorsqu'il ne s'agit pas de polymères, l'éthylèneglycol, le diéthylèneglycol et le propylèneglycol.

Lorsqu'il s'agit d'un polymère hydrophile, on peut citer à titre d'exemple les polyéthers, les polyesters sulfonés, les polyamides sulfonés, ou un mélange de ces polymères.

A titre préférentiel, le composé hydrophile est un polyéther et notamment un poly(oxyde d'éthylène) ou poly(oxyde de propylène).

Le groupe hydrophile noté Y dans la formule (A) est facultatif. En effet, les motifs à fonction amine quaternaire ou protonée peuvent suffire à apporter la solubilité ou l'hydrodispersibilité nécessaire pour ce type de polymère dans une solution aqueuse.

Bien que la présence d'un groupe Y hydrophile soit facultative, on préfère cependant des polyuréthanes associatifs cationiques comportant un tel groupe.

Les dérivés de polyuréthanes associatifs de l'invention peuvent être aussi des polyuréthanes polyéthers non ioniques. Plus particulièrement, lesdits polymères comportent dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, ces polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non ioniques à chaîne grasse peuvent être des copolymères triblocs dont la séquence hydrophile est une chaîne polyoxyéthylénée comportant de 50 à 1000 groupements oxyéthylénés.

Les polyéthers polyuréthanes non ioniques comportent une liaison uréthane entre les séquences hydrophiles, d'où l'origine du nom.

Par extension figurent aussi parmi les polyéthers polyuréthanes non ioniques à chaîne hydrophobe, ceux dont les séquences hydrophiles sont liées aux séquences hydrophobes par d'autres liaisons chimiques.

A titre d'exemples de polyéthers polyuréthanes non ioniques à chaîne hydrophobe utilisables dans l'invention, on peut utiliser aussi le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C12-C14 et le produit ELFACOS T212® à chaîne alkyle en C18 de chez AKZO.

Le produit DW 1206B® de chez ROHM & HAAS à chaîne alkyle en C20 et à liaison uréthane, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemples, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéthers polyuréthanes utilisables décrits auparavant peuvent aussi être choisis parmi ceux décrits dans l'article de G. Fonnum, J. Bakke et Fk. Hansen - Colloid Polym. Sci 271, 380.389 (1993).

Plus particulièrement encore, selon l'invention, on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

La composition peut de même comprendre des polymères dérivés de celluloses associatifs tels que :
- les celluloses cationiques quaternisées modifiées par des groupements comportant au moins une chaîne hydrophobe, tels que les groupes alkyle, arylalkyle, alkylaryle comportant au moins 8 atomes de carbone, ou des mélanges de ceux-ci,
- les hydroxyéthylcelluloses cationiques quaternisées modifiées par des groupements comportant au moins une chaîne hydrophobe, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthylcelluloses quaternisées à chaîne hydrophobe en C8-C30, les produits QUATRISOFT LM 200®, QUATRISOFT LM-X 529-18-A®, QUATRISOFT LM-X 529-18B® (alkyle en C12) et QUATRISOFT LM-X 529-8® (alkyle en C18) commercialisés par la société AMERCHOL et les produits CRODACEL QM®, CRODACEL QL® (alkyle en C12) et CRODACEL QS® (alkyle en C18) commercialisés par la société CRODA.
- les dérivés de cellulose non ioniques tels que les hydroxyéthylcelluloses modifiées par des groupements comportant au moins une chaîne hydrophobe tels que des groupes alkyle, arylalkyle, alkylaryle, ou leurs mélanges, et dans lesquels les groupes alkyle sont de préférence en C8-C22, comme le produit NATROSOL PLUS GRADE 330 CS® (alkyles en C16) vendu par la société AQUALON, ou le produit BERMOCOLL EHM 100® vendu par la société BEROL NOBEL,
- les dérivés de cellulose modifiée par des groupes polyalkylène glycol éther d'alkyl phénol, tel que le produit AMERCELL POLYMER HM-1500® vendu par la société AMERCHOL.

En ce qui concerne les polyvinyllactames associatifs, on peut citer par exemple les polymères notamment décrits dans FR 0101106. Lesdits polymères sont plus particulièrement des polymères cationiques et comprennent :
- a) au moins un monomère de type vinyl lactame ou alkylvinyllactame;
- b) au moins un monomère de structures (a) ou (b) suivantes : 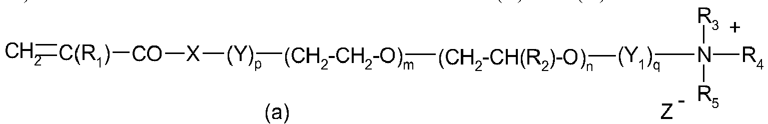 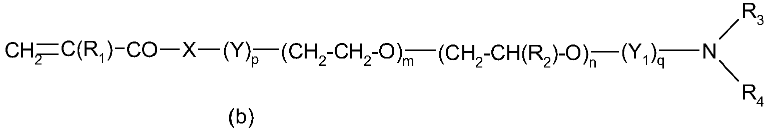 dans lesquelles :
   X désigne un atome d'oxygène ou un radical NR6,
   R1 et R6 désignent, indépendamment l'un de l'autre, un atome d'hydrogène ou un radical alkyl linéaire ou ramifié en C1-C5,
   R2 désigne un radical alkyle linéaire ou ramifié en C1-C4,
   R3, R4 et R5 désignent, indépendamment l'un de l'autre, un atome d'hydrogène, un radical alkyle linéaire ou ramifié en C1-C30 ou un radical de formule (IV) : 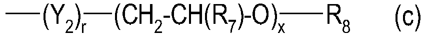
      Y, Y1 et Y2 désignent, indépendamment l'un de l'autre, un radical alkylène linéaire ou ramifié en C2-C16,
      R7 désigne un atome d'hydrogène, ou un radical alkyle linéaire ou ramifié en C1-C4 ou un radical hydroxyalkyle linéaire ou ramifié en C1-C4,
      R8 désigne un atome d'hydrogène ou un radical alkyle linéaire ou ramifié en C1-C30,
      p, q et r désignent, indépendamment l'un de l'autre, soit la valeur zéro, soit la valeur 1,
      m et n désignent, indépendamment l'un de l'autre, un nombre entier allant de 0 à 100,
      x désigne un nombre entier allant de 1 à 100,
      Z désigne un anion d'acide organique ou minéral,
      sous réserve que :
         - l'un au moins des substituants R3, R4, R5 ou R8 désigne un radical alkyle linéaire ou ramifié en C9-C30,
         - si m ou n est différent de zéro, alors q est égal à 1,
         - si m ou n sont égaux à zéro, alors p ou q est égal à 0.

Les polymères poly(vinyllactame) peuvent être réticulés ou non réticulés et peuvent aussi être des polymères blocs.

De préférence le contre ion Z- des monomères de formule (b) est choisi parmi les ions halogénures, les ions phosphates, l'ion méthosulfate, l'ion tosylate.

De préférence R3, R4 et R5 désignent, indépendamment l'un de l'autre, un atome d'hydrogène ou un radical alkyle linéaire ou ramifié en C1-C30.

Plus préférentiellement, le monomère b) est un monomère de formule (b) pour laquelle, encore plus préférentiellement, m et n sont égaux à zéro.

Le monomère vinyllactame ou alkylvinyllactame est de préférence un composé de structure (d) : 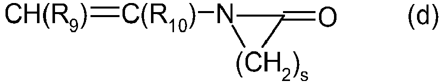 dans laquelle :
s désigne un nombre entier allant de 3 à 6,
R9 désigne un atome d'hydrogène ou un radical alkyle en C1-C5,
R10 désigne un atome d'hydrogène ou un radical alkyle en C1-C5,
sous réserve que l'un au moins des radicaux R9 et R10 désigne un atome d'hydrogène.

Encore plus préférentiellement, le monomère (d) est la vinylpyrrolidone.

Les polymères poly(vinyllactame) peuvent également contenir un ou plusieurs monomères supplémentaires, de préférence cationiques ou non ioniques.

A titre de composés plus particulièrement préférés selon l'invention, on peut citer les terpolymères suivants comprenant au moins :
a)-un monomère de formule (d),
b)-un monomère de formule (a) dans laquelle p=1, q=0, R3 et R4 désignent, indépendamment l'un de l'autre, un atome d'hydrogène ou un radical alkyle en C1-C5 et R5 désigne un radical alkyle en C9-C24 et
c)-un monomère de formule (b) dans laquelle R3 et R4 désignent, indépendamment l'un de l'autre, un atome d'hydrogène ou un radical alkyle en C1-C5.

Encore plus préférentiellement, on met en oeuvre des terpolymères comprenant, en poids, 40 à 95% de monomère (d), 0,1 à 55% de monomère (b) et 0,25 à 50% de monomère (b).

De tels polymères sont notamment décrits dans la demande de brevet WO 00/68282 dont le contenu fait partie intégrante de l'invention.

Comme polymères poly(vinyllactame), on utilise notamment les terpolymères vinylpyrrolidone / diméthylaminopropylméthacrylamide / tosylate de dodécyl diméthylméthacrylamidopropylammonium, les terpolymères vinylpyrrolidone / diméthyl aminopropylméthacrylamide / tosylate de cocoyldiméthylméthacrylamido propyl ammonium, les terpolymères vinylpyrrolidone / diméthylaminopropyl méthacrylamide / tosylate ou chlorure de lauryldiméthylméthacrylamidopropylammonium. Le terpolymère vinylpyrrolidone / diméthylaminopropyl méthacrylamide /chlorure de lauryl diméthylméthacrylamidopropylammonium est proposé dans l'eau à 20% par la Société ISP sous la dénomination STYLEZE W20.

Les dérivés de polyvinyllactames associatifs de l'invention peuvent être aussi des copolymères non ioniques de vinylpyrrolidone et de monomères hydrophobes à chaîne hydrophobe dont on peut citer à titre d'exemple :
- les produits ANTARON V216® ou GANEX V216® (copolymère vinylpyrrolidone / hexadécène) vendu par la société I.S.P.
- les produits ANTARON V220® ou GANEX V220® (copolymère vinylpyrrolidone / eicosène) vendu par la société I.S.P.

Parmi les dérivés de polyacides insaturés associatifs on peut citer ceux comportant au moins un motif hydrophile de type acide carboxylique insaturé oléfinique, et au moins un motif hydrophobe de type ester d'alkyl (C10-C30) non éthoxylé d'acide carboxylique insaturé.

Ces polymères sont notamment choisis parmi ceux dont le motif hydrophile de type acide carboxylique insaturé oléfinique correspond au monomère de formule (e) suivante : 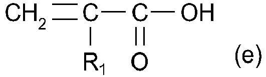 dans laquelle, R1 désigne H ou CH3 ou C2H5, c'est-à-dire des motifs acide acrylique, acide méthacrylique ou acide éthacrylique, et dont le motif hydrophobe de type ester d'alkyl (C10-C30) d'acide carboxylique insaturé correspond au monomère de formule (f) suivante : 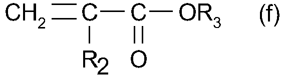 dans laquelle, R2 désigne H ou CH3 ou C2H5 (c'est-à-dire des motifs acrylates, méthacrylates ou éthacrylates) et de préférence H (motifs acrylates) ou CH3 (motifs méthacrylates), R3 désignant un radical alkyle en C10-C30, et de préférence en C12-C22.

Des esters d'alkyle (C10-C30) d'acides carboxyliques insaturés comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères anioniques de ce type sont par exemple décrits et préparés, selon les brevets US-3 915 921 et 4 509 949.

Dans ce type de polymères associatifs anioniques, on utilise plus particulièrement des polymères formés à partir d'un mélange de monomères comprenant :
essentiellement de l'acide acrylique,
un ester de formule (f) décrite ci-dessus et dans laquelle R2 désigne H ou CH3, R3 désignant un radical alkyle ayant de 12 à 22 atomes de carbone,
et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères associatifs anioniques, on préfère ceux constitués de 95 à 60% en poids d'acide acrylique (motif hydrophile), de 4 à 40% en poids d'acrylate d'alkyles en C10-C30 (motif hydrophobe), et de 0 à 6% en poids de monomère polymérisable réticulant, ou bien ceux constitués de 98 à 96% en poids d'acide acrylique (motif hydrophile), de 1 à 4% en poids d'acrylate d'alkyles en C10-C30 (motif hydrophobe), et de 0,1 à 0,6% en poids de monomère polymérisable réticulant tel que ceux décrits précédemment.

Parmi lesdits polymères ci-dessus, on préfère tout particulièrement les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.

En ce qui concerne les polymères épaississants du type des aminoplaste-éther, on désigne tout produit issu de la condensation d'un aldéhyde avec une amine ou un amide, ainsi que toute unité structurale formée d'un résidu aminoplaste et d'un résidu hydrocarboné divalent lié au résidu aminoplaste par une liaison éther.

Les polymères à squelette aminoplaste-éther sont choisis de préférence parmi ceux contenant au moins un motif de structure (g) suivante : 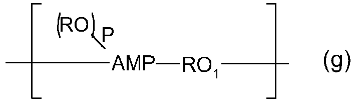 dans laquelle :
- AMP est un résidu aminoplaste avec des unités alkylènes (ou alkyle divalent),
- R désigne un atome d'hydrogène, un radical alkyle C1-C4 ou un radical acyle C1-C4,
- RO1 est un résidu alkylène-oxy divalent,
- p désigne un nombre entier positif,
- le ou les groupements OR étant liés aux unités alkylènes du résidu AMP.

De préférence, les polymères à squelette aminoplaste-éther sont choisis parmi ceux contenant au moins un motif de structure (h) suivante 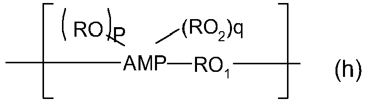 dans laquelle :
- AMP, R, RO1 et p ont la même signification que précédemment,
- RO2 est un groupe différent de RO lié à AMP au moyen d'un hétéroatome et comprenant au moins deux atomes de carbone, et,
- q est un nombre entier positif.

Plus préférentiellement encore, les polymères correspondent aux formules (III) et (III)bis suivantes : 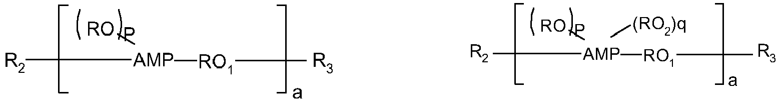 dans lesquelles :
- AMP, R, RO1, RO2, p et q ont la même signification que précédemment,
- R2 ou R3, identiques ou différents représentent un groupe terminal pouvant désigner un atome d'hydrogène, un groupement RO1H, un groupement RO2H, un groupement AMP(OR)p ou tout groupement monofonctionnel tel que alkyle, cycloalkyle, aryle, aralkyle, alkylaryle, alkyloxyalkyle, aryloxyalkyle, cycloalkoxyalkyle,
- a étant un nombre supérieur à 1 et de préférence supérieur à 2.

Les résidus aminoplastes porteurs de leurs groupements OR intégrés dans les polymères peuvent être choisis de manière non limitative parmi les structures (1) à (12) suivantes : 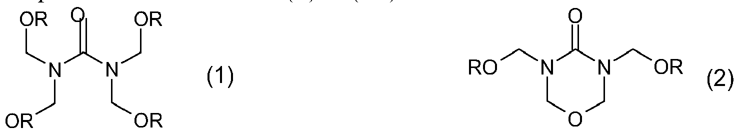 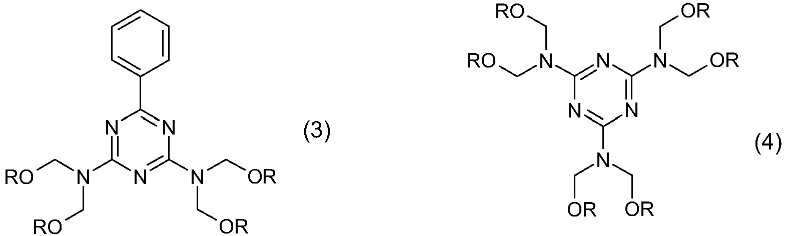 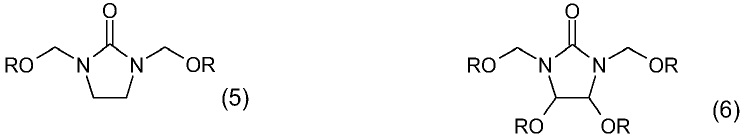 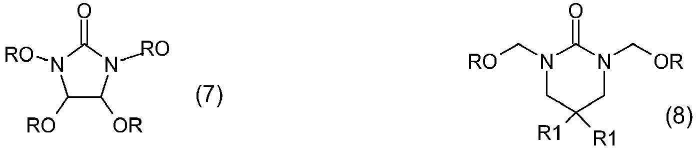 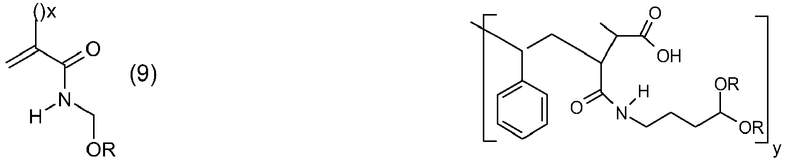 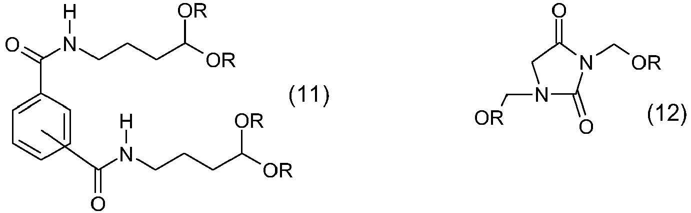 dans lesquelles :
- R a la même signification que précédemment,
- R1 désigne alkyle C1-C4,
- y est un nombre au moins égal à 2,
- x désigne 0 ou 1.

De préférence, le ou les résidus aminoplastes porteurs de leurs groupements OR sont choisis parmi ceux de structure (13) suivante : 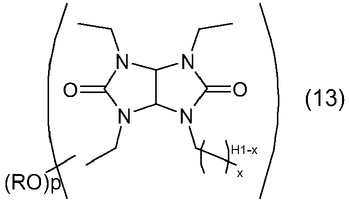 dans laquelle R, p , et x ont les mêmes significations que précédemment.

Les résidus alkylène-oxy divalents sont de préférence ceux correspondants aux diols de formule générale (14) suivante :

HO-(ZO)y-(Z1(Z2O)w)t-(Z'O)y'-Z3OH (14),

- y et y' étant des nombres allant de 0 à 1000,
- t et w étant des nombres allant de 0 à 10,
- Z, Z', Z2 et Z3 sont des radicaux alkylène en C2-C4 et de préférence des radicaux -CH2-CH(Z4)- et -CH2-CH(Z4)-CH2-,
- Z1 étant un radical linéaire ou cyclique, ramifié ou non, aromatique ou non, comportant ou non un ou plusieurs hétéroatomes et possédant de 1 à 40 atomes de carbone,
- Z4 désignant un atome d'hydrogène ou un radical alkyle en C1-C4 ou un radical acyle en C1-C3 étant entendu qu'au moins un des radicaux Z4 des radicaux Z, Z', Z2 et Z3 est différent d'un radical acyle.

De préférence Z4 désigne un atome d'hydrogène ou un radical méthyle.

Encore plus préférentiellement t=0 et Z, Z' et Z3 désignent - CH2CH2-, et l'un au moins de y ou y' est différent de 0. Les composés de formule (14) sont alors des polyéthylèneglycols.

Les polymères aminoplaste éther de formule (g) contiennt au moins une chaîne linéaire ou cyclique comportant au moins 8 atomes de carbone, saturée ou insaturée, et sont en particulier décrits dans le brevet US 5 914 373 auquel on pourra se référer pour plus de détails.

Comme polymères à squelette aminoplaste-éther de formule (g), on peut en particulier citer les produits Pure-Thix® L [PEG-180/Octoxynol-40/TMMG Copolymer (Nom INCI)], Pure-Thix M® [PEG-180/Laureth-50/TMMG Copolymer (Nom INCI)], Pure-Thix® HH [Polyether-1 (Nom INCI)] ; Pure Thix TX-1442® [copolymère PEG-18 / dodoxynol-5 / PEG-25 tristyrylphénol / tétraméthoxy méthyl glycolurile] proposés par la société Süd-Chemie.

Les polymères épaississants entrant comme ingrédient dans la composition selon l'invention peuvent aussi être choisis parmi les polymères associatifs comportant au moins un monomère à insaturation éthylénique à groupement sulfonique, sous forme libre ou partiellement ou totalement neutralisée et comprenant au moins une partie hydrophobe.

De façon préférentielle, lesdits polymères sont neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés.

Ces polymères associatifs peuvent être ou non réticulés, et de préférence sont des polymères réticulés. Dans ce cas, les agents réticulants provenant d'au moins un monomère ayant au moins deux insaturation éthyléniques (double liaison carbone-carbone).

Les monomères de réticulation ayant au moins deux insaturations éthyléniques sont choisis par exemple parmi l'éther diallylique, le triallylcyanurate, le diallylmaléate, le (méth)acrylate d'allyle, le dipropylèneglycol-diallyléther, les polyglycol-diallyléthers, le triéthylèneglycol-divinyléther, l'hydroquinone-diallyl-éther, le tétrallyl-oxéthanoyle, le di(méth)acrylate de tétra- ou di-éthylèneglycol, la triallylamine, la tétraallyléthylènediamine le triméthylolpropane-diallyléther, le triméthylolpropane triacrylate, le méthylène-bis(méth)acrylamide ou le divinylbenzène, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

On utilise plus particulièrement le méthylène-bis-acrylamide, le méthacrylate d'allyle ou le triméthylol propane triacrylate. Le taux de réticulation varie en général de 0,01 à 10% en mole, par rapport au polymère.

Les monomères à insaturation éthylénique à groupement sulfonique sont choisis notamment parmi l'acide vinylsulfonique, l'acide styrènesulfonique, les acides (méth)acrylamido(C1-C22)alkylsulfoniques, les acides N-(C1-C22)alkyl(méth)acrylamido(C1-C22)alkylsulfoniques comme l'acide undécyl-acrylamido-méthane-sulfonique ainsi que leurs formes partiellement ou totalement neutralisées.

Plus particulièrement, on peut utiliser les acides (méth)acrylamido(C1-C22) alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique ainsi que leurs formes partiellement ou totalement neutralisées.

On utilise de préférence l'acide 2-acrylamido-2-méthylpropane-sulfonique (AMPS) ainsi que ses formes partiellement ou totalement neutralisées.

Les polymères amphiphiles présents dans la composition selon l'invention peuvent aussi être choisis parmi les polymères amphiphiles statistiques d'AMPS modifiés par réaction avec une n-monoalkylamine ou une di-n-alkylamine en C6-C22, et tels que ceux décrits dans la demande WO00/31154.

Les monomères hydrophobes qui constituent la partie hydrophobe du polymère sont choisis de préférence parmi les acrylates ou les acrylamides de formule (k) suivante : 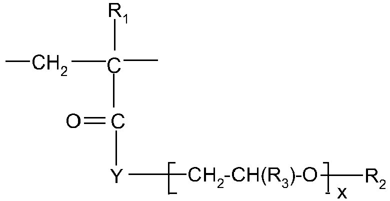 dans laquelle R1 et R3, identiques ou différents, désignent un atome d'hydrogène ou un radical alkyle linéaire ou ramifié en C1-C6 (de préférence méthyle) ; Y désigne O ou NH ; R2 désigne un radical hydrocarboné hydrophobe tel que défini auparavant ; x désigne un nombre de moles d'oxyde d'alkylène et varie de 0 à 100.

Le radical R2 est choisi avantageusement parmi les radicaux alkyles en C6-C18 linéaires (par exemple n-hexyle, n-octyle, n-décyle, n-hexadécyle, n-dodécyle), ramifiés ou cycliques (par exemple cyclododécane (C12) ou adamantane (C10)) ; les radicaux alkylperfluorés en C6-C18 (par exemple le groupement de formule - (CH2)2-(CF2)9-CF3) ; le radical cholestéryle (C27) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle.

Selon une forme particulière de l'invention, le monomère de formule (k) comporte au moins un motif oxyde d'alkylène (x ≥1) et de préférence une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés varie en général de 3 à 100 et plus préférentiellement de 3 à 50 et encore plus préférentiellement de 7 à 25.

Les copolymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou poly-alkylèneglycols, les (méth)acrylamides, la vinylpyrrolidone, l'anhydride maléique, l'acide itaconique ou l'acide maléique ou les mélanges de ces composés.

Ces copolymères sont décrits notamment dans les documents EP750899, US 5089578, les publications de Yotaro Morishima suivantes : «Self-assembling amphiphilic polyelectrolytes and their nanostructures - Chinese Journal of Polymer Science Vol. 18, N°40, (2000), 323-336.» ; «Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704» ; «Solution properties of miscelle networks formed by non-ionic moieties covalently bound to an polyelectrolyte: salt effects on rheological behaviour- Langmuir, 2000, Vol.16, N°12, 5324-5332» ; «Stimuli responsive amphiphilic copolymers of sodium 2-(acrylamido)-2-methylpropanesulfonate and associative macromonomers - Polym. Preprint, Div. Polym. Chem. 1999, 40(2), 220-221».

La répartition des monomères dans le copolymère peut être statistique ou bloc.

Parmi ces polymères de ce type, on peut citer plus spécialement :
- les copolymères réticulés ou non réticulés, neutralisés ou non, comportant de 15 à 60% en poids de motifs AMPS et de 40 à 85% en poids de motifs (C8-C16)alkyl(méth)acrylamide ou de motifs (C8-C16)alkyl(méth)acrylate par rapport au polymère, tels que ceux décrits dans la demande EP-A750 899 ;
- les terpolymères comportant de 10 à 90% en mole de motifs acrylamide, de 0,1 à 10% en mole de motifs AMPS et de 5 à 80% en mole de motifs n-(C6-C18)alkylacrylamide, tels que ceux décrits dans le brevet US- 5089578.
- les copolymères d'AMPS totalement neutralisé et de méthacrylate de dodécyle ainsi que les copolymères d'AMPS et de n-dodécylméthacrylamide non-réticulés et réticulés, tels que ceux décrits dans les articles de Morishima cités ci-dessus.

On citera plus particulièrement les copolymères constitués de motifs AMPS de formule (XXX) suivante : 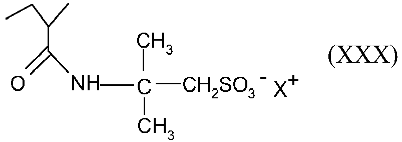 dans laquelle X+ a la même définition que précédemment,
et de motifs de formule (XXXI) suivante : 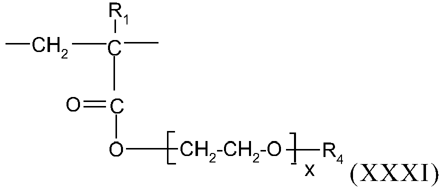 dans laquelle x désigne un nombre entier variant de 3 à 100, de préférence de 5 à 80 et plus préférentiellement de 7 à 25 ; R1 a la même signification que celle indiquée ci-dessus dans la formule (j) et R4 désigne un alkyle linéaire ou ramifié en C6-C22 et plus préférentiellement en C10-C22.

Les polymères particulièrement préférés sont ceux pour lesquels x = 25, R1 désigne méthyle et R4 représente n-dodécyle ; ils sont décrits dans les articles de Morishima mentionnés ci-dessus.

Les polymères pour lesquels X+ désigne sodium ou ammonium sont plus particulièrement préférés.

Les polymères de la gamme Genapol® de la société Hoechst/Clariant peuvent être employés dans la composition selon l'invention.

La concentration agents d'ajustement additionnels associatif(s) ou non, présents dans la composition selon l'invention peut varier entre 0,01 et 10% en poids, plus particulièrement entre 0,1 à 5% en poids, par rapport au poids de la composition, et de manière encore plus avantageuse, entre 0,5 et 5 % en poids par rapport au poids de la composition.

Les compositions cosmétiques conformes à l'invention peuvent se présenter sous forme de crème, de mousse ou de gel. De préférence, la composition selon l'invention se présente sous forme d'un gel. Plus préférentiellement, ce gel présente une viscosité supérieure à 200 cps à 25 °C à un taux de cisaillement de 1s⁻¹. Tout particulièrement, cette viscosité est comprise entre 500 et 500000 cps à 25 °C, de préférence entre 500 et 100000 cps à 25 °C, et encore plus préférentiellement entre 500 et 50000 cps à 25 °C à un taux de cisaillement de 1s⁻¹. Elle peut être mesurée à l'aide d'un viscosimètre de type comeplan.

La composition cosmétique selon l'invention peut comprendre en outre un ou plusieurs adjuvants cosmétiques choisis parmi les agents tensioactifs anioniques ou non ioniques, les agents conditionneurs de type esters, les agents anti-mousse, les agents hydratants, les agents émollients, les plastifiants, les filtres solaires hydrosolubles et liposolubles, siliconés ou non siliconés, les colorants permanents ou temporaires, les parfums, les peptisants, les conservateurs, les céramides, et pseudo-céramides, les vitamines et les provitamines dont le panthénol, les protéines, les agents séquestrants, les agents solubilisants, les agents alcanisants, les agents acidifiants, les agents anti-corrosion, les corps gras non siliconés tels que les huiles végétales, animales, minérales et synthétiques, et les cires, les agents réducteurs ou anti-oxydants, les agents oxydants, les charges minérales, les paillettes.

L'homme du métier veillera à choisir les éventuels adjuvants et leur quantité de manière à ce qu'ils ne nuisent pas aux propriétés des compositions de la présente invention.

De préférence, le ou les adjuvants cosmétiques sont présents à une concentration allant de 0,001 à 50 % en poids par rapport au poids total de la composition.

Par milieu cosmétiquement acceptable, on entend un milieu compatible avec les matières kératiniques et en particulier les cheveux.

Le milieu cosmétiquement acceptable peut être un milieu alcoolique, aqueux ou hydroalcoolique. Ainsi le milieu peut notamment être constitué uniquement par de l'eau ou d'un monoalcool ou par un mélange d'eau et d'un ou de plusieurs monoalcools cosmétiquement acceptables tels que les alcools inférieurs en C₁-C₄, les éthers de polyols présentant un hydroxyle libre et leurs mélanges. De préférence, l'alcool est l'éthanol.

La présente invention concerne aussi un procédé de traitement cosmétique des cheveux, par exemple de coiffage, qui consiste à appliquer une quantité efficace d'une composition décrite ci-dessus, sur les cheveux secs ou humides, à rincer ou non après un éventuel temps de pose ou après un éventuel séchage.

La présente invention concerne également l'utilisation d'une composition cosmétique pour la fixation des cheveux.

Les exemples suivants sont donnés à titre illustratif et non limitatif de la présente invention.

### EXEMPLE

Les compositions selon l'invention ont été préparées :

| Nom INCI | A | B | C | D | E | F |
|---|---|---|---|---|---|---|
| Acrylates/steareth - 20/ methacrylate copolymer (Aculyn 22® de Rohm and Haas) | 0,33 | 1,32 | 0,66 | 0,99 | 0,99 | 1,98 |
| Polyvinylalcohol (Kuraray) | | | 1 | | | |
| Polystyrene (Synthron) | | | | 1 | | |
| PVP (ISP) | 1 | 6,5 | | | 1 | 1 |
| PEG-90M (Dow Chemical) | | | | | 0,5 | 0,1 |
| PPG-3methylether (Dow Chemical) | | | | 1 | | |
| PEG-8 (Dow Chemical) | | 15 | | 0,5 | | |
| Dipropyleneglycol (BASF) | | | | | 2 | |
| Propyleneglycol (BASF) | | | 5 | | | |
| Glycerin (Cognis) | 1 | | | | | 5 |
| Carbomer (Noveon) | 0,5 | | | | | |
| Hydroxypropylguar (Rhodia) | | | | | 0,1 | |
| Acrylate/C10-C30 alkylacrylate crosspolymer (Noveon) | | | 0,3 | | | |
| Amodimethicone (and) trideceth-6 (and) cetrimonium chloride (and) dimethyltin dineodecylester (Dow Corning) | | 0,8 | | | | |
| Polyquaternium-4 (National Starch) | | | | 0,5 | | |
| Polyquaternium-11 (ISP) | | | | | 1 | |
| AMP-Acrylates/allyl methacrylate copolymer (Noveon) | | | 2 | | | |
| PEG-14 Dimethicone (Degussa) | | | | | | 1 |
| Benzohenone-4 | 0,1 | | 0,05 | | | 0,1 |
| Disodium EDTA (BASF) | 0,2 | | 0,1 | | | 0,2 |
| Conservateurs | 0,3 | 0,3 | 0,3 | 0,3 | 0,3 | 0,3 |
| Parfum | 0,3 | 0,3 | 0,3 | 0,3 | 0,3 | 0,3 |
| Triethanolamine | QSP pH 7,5 | | | | | |
| Eau | Qsp 100g | | | | | |

Ces compositions sont appliquées sur les cheveux en mode non rincé. Ces compositions permettent un maintien durable de la forme de la coiffure.

## Revendications

1. Composition cosmétique pour le traitement des fibres kératiniques, en particulier des fibres kératiniques humaines telles que les cheveux, comprenant, dans un milieu cosmétiquement acceptable :
- au moins un terpolymère comportant parmi ses monomères au moins un acide carboxylique à insaturation α,β-monoéthylénique ; au moins un ester d'acide carboxylique à insaturation α,β-monoéthylénique et au moins un ester d'acide carboxylique à insaturation α,β-monoéthylénique et d'alcool gras oxyalkyléné ;
- au moins un polymère fixant choisi parmi les homopolymères fixants non-ioniques, le rapport entre les quantités exprimées en pourcentage en poids par rapport au poids total de la composition du terpolymère sur l'homopolymère fixant non-ionique étant supérieur ou égal à 0,2,
- au moins un polyol différent de l'homopolymère fixant non-ionique, et le rapport entre les quantités exprimées en pourcentage en poids par rapport au poids total de la composition du terpolymère sur le polyol étant inférieur à 1, et
- si la composition comprend de plus un polymère fixant anionique, alors le terpolymère se trouve en une quantité suffisante pour obtenir une viscosité supérieure à 50 cps à 25°C à un taux de cisaillement de 1s⁻¹.

2. Composition cosmétique selon la revendication 1, **caractérisée par le fait que** le terpolymère comporte comme monomère au moins un acide carboxylique à insaturation α,β-monoéthylénique en C₁-C₁₀; au moins un ester d'acide carboxylique à insaturation α,β-monoéthylénique en C₃-C₁₀ et d'alcool en C₁-C₄ et au moins un ester d'acide carboxylique à insaturation α,β-monoéthylénique d'alcool gras oxyalkyléné en C₁₀-C₂₆.

3. Composition cosmétique selon la revendication 2, **caractérisée par le fait que** le terpolymère est un copolymère d'acide méthacrylique ; d'acrylate d'éthyle et de méthacrylate de stéaryle oxyalkyléné.

4. Composition cosmétique selon l'une quelconque des revendications 1 à 3, **caractérisée par le fait que** la masse moléculaire en poids des terpolymères est comprise entre 500 et 20 000 000, de préférence entre 200 000 et 2 000 000 et encore plus préférentiellement entre 400 000 et 800 000.

5. Composition cosmétique selon l'une quelconque des revendications 1 à 4, **caractérisée par le fait que** le(s) terpolymère(s) est ou sont présents dans la composition cosmétique en une quantité variant de 0,05 à 30 % en poids, de préférence en une quantité variant de 0,1 à 15 % en poids et encore plus préférentiellement en une quantité variant de 0,2 à 10 % en poids, par rapport au poids total de la composition.

6. Composition cosmétique selon l'une quelconque des revendications précédentes, **caractérisée par le fait que** l'homopolymère fixant non ionique est choisi parmi :
- les homopolymères d'acétate de vinyle ou d'alcool de vinyle;
- les homopolymères de styrène ;
- les polyalkyloxazolines ;
- les homopolymères de vinyllactame ;
- les homopolymères d'acrylates d'alkyle et les homopolymères de méthacrylates d'alkyle.

7. Composition cosmétique selon la revendication précédente, **caractérisée par le fait que** le poids moléculaire en nombre de l'homopolymère fixant non ionique est supérieur à 350.

8. Composition cosmétique selon l'une quelconque des revendications précédentes, **caractérisée par le fait que** le ou les homopolymères fixants non-ioniques sont présents dans la composition dans une quantité variant de 0,1 à 30 %, de préférence en une quantité variant de 0,2 à 20 %, et encore plus préférentiellement dans une quantité variant de 0,5 à 10 % en poids, par rapport au poids total de la composition.

9. Composition cosmétique selon l'une quelconque des revendications précédentes, **caractérisée par le fait que** les polyols sont de formule (II) : 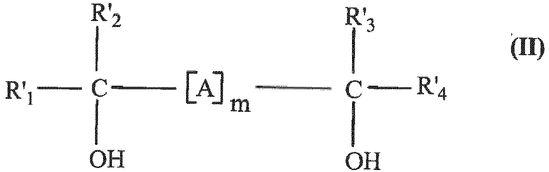 dans laquelle R'₁, R'₂, R'₃, R'₄ désignent indépendamment l'un de l'autre un atome d'hydrogène, un radical alkyle en C₁-C₆ ou un radical mono ou polyhydroxyalkyle en C₁-C₆,
A désigne un radical alkylène linéaire ou ramifié contenant de 1 à 18 atomes de carbone, ce radical comprend de 0 à 9 atomes d'oxygène mais pas de groupement hydroxyle,
m désigne 0 ou 1.

10. Composition cosmétique selon la revendication précédente, **caractérisée par le fait que** lorsque m vaut 0, alors le polyol est choisi parmi le 1,2,3-propanetriol, le pinacol (2,3-diméthyl 2,3-butanediol), le 1,2,3-butanetriol, le 2,3-butanediol et le sorbitol.

11. Composition cosmétique selon la revendication 9, **caractérisée par le fait que** lorsque m vaut 1, alors R'₁, R'₂, R'₃, R'₄ désignent, indépendamment l'un de l'autre, un atome d'hydrogène ou un radical alkyle en C₁-C₆.

12. Composition cosmétique selon la revendication précédente, **caractérisée par le fait que** le polyol est choisi parmi le PEG-6 ou le PEG-8.

13. Composition cosmétique selon la revendication 9, **caractérisée par le fait que** lorsque m vaut 1, alors R'₁, R'₂, R'₃, R'₄ désignent, indépendamment l'un de l'autre, un atome d'hydrogène ou un radical alkyle en C₁-C₆, le poids moléculaire du polyol étant inférieur à 200.

14. Composition cosmétique selon la revendication précédente, **caractérisée par le fait que** le polyol est choisi parmi le 3-méthyl-1,3,5-pentanetriol, le 1,2,4-butanetriol, le 1,5-pentanediol, le 2-méthyl-1,3 propanediol, le 1,3-butanediol, le 3-méthyl-1,5-pentanediol, le néopentylglycol (2,2-diméthyl-1,3-propanediol), l'isoprène glycol (3-méthyl-1,3-butanediol) et l'hexylèneglycol (2-méthyl-2,4-pentanediol) et de manière encore plus préférée l'hexylèneglycol, le néopentylglycol et le 3-méthyl-1,5-pentanediol.

15. Composition cosmétique selon l'une quelconque des revendications 9 à 14, **caractérisée par le fait que** le poids moléculaire du polyol est compris entre 75 et à 350.

16. Composition cosmétique selon la revendication 15, **caractérisée par le fait que** le poids moléculaire du polyol est compris entre 90 et à 350.

17. Composition cosmétique selon la revendication 16, **caractérisée par le fait que** le poids moléculaire du polyol est compris entre 95 et à 350.

18. Composition cosmétique selon l'une quelconque des revendications précédentes, **caractérisée par le fait que** le ou les polyols peuvent être présents dans la composition à une teneur comprise entre 0,1 et 30 % de préférence entre 0,5 et 20 %, et encore plus préférentiellement entre 1 et 15 % en poids du poids total de la composition cosmétique.

19. Composition cosmétique selon l'une quelconque des revendications précédentes, **caractérisée par le fait qu'**elle comprend en outre au moins une silicone.

20. Composition cosmétique selon l'une quelconque des revendications précédentes, **caractérisée par le fait qu'**elle comprend en outre au moins un polymère fixant additionnel ionique ou non-ionique.

21. Composition cosmétique selon l'une quelconque des revendications précédentes, **caractérisée par le fait qu'**elle présente une viscosité supérieure à 200 cps à 25 °C à un taux de cisaillement de 1s⁻¹.

22. Composition cosmétique selon la revendication 21, **caractérisée par le fait qu'**elle présente une viscosité comprise entre 500 et 500000 cps à 25 °C, de préférence entre 500 et 100000 cps à 25 °C, et encore plus préférentiellement entre 500 et 50000 cps à 25 °C à un taux de cisaillement de 1s⁻¹.

23. Composition cosmétique selon l'une quelconque des revendications précédentes, **caractérisée par le fait qu'**elle comprend en outre au moins un adjuvant cosmétique choisi parmi les agents tensioactifs anioniques ou non ioniques, les agents conditionneurs de type esters, les agents anti-mousse, les agents hydratants, les agents émollients, les plastifiants, les filtres solaires hydrosolubles et liposolubles, siliconés ou non siliconés, les colorants permanents ou temporaires, les parfums, les peptisants, les conservateurs, les céramides, et pseudo-céramides, les vitamines et les provitamines dont le panthénol, les protéines, les agents séquestrants, les agents solubilisants, les agents alcanisants, les agents acidifiants, les agents anti-corrosion, les corps gras non siliconés tels que les huiles végétales, animales, minérales et synthétiques et les cires, les agents réducteurs ou anti-oxydants, les agents oxydants, les charges minérales, les paillettes.

24. Composition cosmétique selon l'une quelconque des revendications précédentes, **caractérisée par le fait que** le milieu cosmétiquement acceptable est un milieu aqueux, alcoolique ou hydroalcoolique.

25. Procédé de traitement cosmétique **caractérisée par le fait qu'**il comprend l'application d'une composition cosmétique selon l'une quelconque des revendications 1 à 24, en particulier sur les cheveux.

26. Procédé de traitement cosmétique selon la revendication 25, **caractérisée par le fait que** l'application de ladite composition n'est pas suivie par un rinçage.

27. Utilisation d'une composition cosmétique selon l'une quelconque des revendications 1 à 24 pour la fixation des cheveux.

28. Utilisation d'une composition cosmétique selon l'une quelconque des revendications 1 à 24 pour le soin des cheveux.

## Patentansprüche

1. Kosmetische Zusammensetzung zur Behandlung von Keratinfasern, insbesondere menschlichen Keratinfasern wie dem Haar, die in einem kosmetisch unbedenklichen Medium Folgendes umfasst:
- mindestens ein Terpolymer, das unter seinen Monomeren mindestens eine α,β-monoethylenisch ungesättigte Carbonsäure, mindestens einen Ester einer α,β-monoethylenisch ungesättigten Carbonsäure und mindestens einen Ester einer α,β-monoethylenisch ungesättigten Carbonsäure und eines oxyalkylenierten Fettalkohols umfasst;
- mindestens ein fixierendes Polymer, das aus nichtionischen fixierenden Homopolymeren ausgewählt ist, wobei das Verhältnis zwischen den in Gewichtsprozent, bezogen auf das Gesamtgewicht der Zusammensetzung, ausgedrückten Mengen des Terpolymers und dem nichtionischen fixierenden Homopolymer größer oder gleich 0,2 ist,
- mindestens ein Polyol, das von dem nichtionischen fixierenden Homopolymer verschieden ist, und wobei das Verhältnis zwischen den in Gewichtsprozent, bezogen auf das Gesamtgewicht der Zusammensetzung, ausgedrückten Mengen des Terpolymers und dem Polyol kleiner als 1 ist,
- das Terpolymer dann, wenn die Zusammensetzung außerdem ein anionisches fixierendes Polymer enthält, in einer zum Erhalt einer Viskosität von mehr als 50 cps bei 25 °C bei einer Scherrate von 1 s⁻¹ ausreichenden Menge vorliegt.

2. Kosmetische Zusammensetzung nach Anspruch 1, **dadurch gekennzeichnet, dass** das Terpolymer als Monomer mindestens eine α,β-monoethylenisch ungesättigte Carbonsäure mit 1 bis 10 C-Atomen, mindestens einen Ester einer α,β-monoethylenisch ungesättigten Carbonsäure mit 3 bis 10 C-Atomen und mindestens eines Alkohols mit 1 bis 4 C-Atomen und mindestens einen Ester einer α,β-monoethylenisch ungesättigten Carbonsäure und eines oxyalkylenierten Fettalkohols mit 10 bis 26 C-Atomen umfasst.

3. Kosmetische Zusammensetzung nach Anspruch 2, **dadurch gekennzeichnet, dass** es sich bei dem Terpolymer um ein Copolymer von Methacrylsäure, Ethylacrylat und oxyalkyleniertem Stearylmethacrylat handelt.

4. Kosmetische Zusammensetzung nach einem der Ansprüche 1 bis 3, **dadurch gekennzeichnet, dass** die gewichtsmittlere Molmasse der Terpolymere zwischen 500 und 20.000.000, vorzugsweise zwischen 200.000 und 2.000.000 und noch weiter bevorzugt zwischen 400.000 und 800.000 liegt.

5. Kosmetische Zusammensetzung nach einem der Ansprüche 1 bis 4, **dadurch gekennzeichnet, dass** das Terpolymer bzw. die Terpolymere in der kosmetischen Zusammensetzung in einer Menge im Bereich von 0,05 bis 30 Gew.-%, vorzugsweise in einer Menge im Bereich von 0,1 bis 15 Gew.-% und noch weiter bevorzugt in einer Menge im Bereich von 0,2 bis 10 Gew.-%, bezogen auf das Gesamtgewicht der Zusammensetzung, vorliegt bzw. vorliegen.

6. Kosmetische Zusammensetzung nach einem der vorhergehenden Ansprüche, **dadurch gekennzeichnet, dass** das nichtionische fixierende Homopolymer ausgewählt ist aus:
- Homopolymeren von Vinylacetat oder Vinylalkohol;
- Homopolymeren von Styrol;
- Polyalkyloxazolinen;
- Homopolymeren von Vinyllactam;
- Homopolymeren von Alkylacrylaten und Homopolymeren von Alkylmethacrylaten.

7. Kosmetische Zusammensetzung nach dem vorhergehenden Anspruch, **dadurch gekennzeichnet, dass** das zahlenmittlere Molekulargewicht des nichtionischen fixierenden Homopolymer mehr als 350 beträgt.

8. Kosmetische Zusammensetzung nach einem der vorhergehenden Ansprüche, **dadurch gekennzeichnet, dass** das nichtionische fixierende Homopolymer bzw. die nichtionischen fixierenden Homopolymere in der Zusammensetzung in einer Menge im Bereich von 0,1 bis 30 Gew.-%, vorzugsweise in einer Menge im Bereich von 0,2 bis 20 Gew.-% und noch weiter bevorzugt in einer Menge im Bereich von 0,5 bis 10 Gew.-%, bezogen auf das Gesamtgewicht der Zusammensetzung, vorliegt bzw. vorliegen.

9. Kosmetische Zusammensetzung nach einem der vorhergehenden Ansprüche, **dadurch gekennzeichnet, dass** die Polyole die Formel (II) aufweisen: 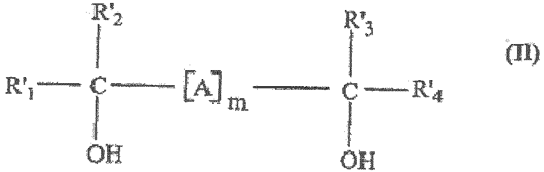 wobei R'₁, R'₂, R'₃ und R'₄ unabhängig voneinander für ein Wasserstoffatom, einen C₁-C₆-Alkylrest oder einen C₁-C₆-Mono- oder Polyhydroxyalkylrest stehen, A für einen linearen oder verzweigten Alkylrest mit 1 bis 18 Kohlenstoffatomen steht, wobei dieser Rest 0 bis 9 Sauerstoffatome, aber keine Hydroxylgruppe umfasst,
m für 0 oder 1 steht.

10. Kosmetische Zusammensetzung nach dem vorhergehenden Anspruch, **dadurch gekennzeichnet, dass** dann, wenn m gleich 0 ist, das Polyol aus 1,2,3-Propantriol, Pinacol (2,3-Dimethyl-2,3-butandiol), 1,2,3-Butantriol, 2,3-Butandiol und Sorbitol ausgewählt ist.

11. Kosmetische Zusammensetzung nach Anspruch 9, **dadurch gekennzeichnet, dass** dann, wenn m gleich 1 ist, R'₁, R'₂, R'₃ und R'₄ unabhängig voneinander für ein Wasserstoffatom oder einen C₁-C₆-Alkylrest stehen.

12. Kosmetische Zusammensetzung nach dem vorhergehenden Anspruch, **dadurch gekennzeichnet, dass** das Polyol aus PEG-6 oder PEG-8 ausgewählt ist.

13. Kosmetische Zusammensetzung nach Anspruch 9, **dadurch gekennzeichnet, dass** dann, wenn m gleich 1 ist, R'₁, R'₂, R'₃ und R'₄ unabhängig voneinander für ein Wasserstoffatom oder einen C₁-C₆-Alkylrest stehen, wobei das Molekulargewicht des Polyols weniger als 200 beträgt.

14. Kosmetische Zusammensetzung nach dem vorhergehenden Anspruch, **dadurch gekennzeichnet, dass** das Polyol aus 3-Methyl-1,3,5-pentantriol, 1,2,4-Butantriol, 1,5-Pentandiol, 2-Methyl-1,3-propandiol, 1,3-Butandiol, 3-Methyl-1,5-pentandiol, Neopentylglykol (2,2-Dimethyl-1,3-propandiol), Isoprenglykol (3-Methyl-1,3-butandiol) und Hexylenglykol (2-Methyl-2,4-pentandiol) und noch weiter bevorzugt aus Hexylenglykol, Neopentylglykol und 3-Methyl-1,5-pentandiol ausgewählt ist.

15. Kosmetische Zusammensetzung nach einem der Ansprüche 9 bis 14, **dadurch gekennzeichnet, dass** das Molekulargewicht des Polyols zwischen 75 und 350 liegt.

16. Kosmetische Zusammensetzung nach Anspruch 15, **dadurch gekennzeichnet, dass** das Molekulargewicht des Polyols zwischen 90 und 350 liegt.

17. Kosmetische Zusammensetzung nach Anspruch 16, **dadurch gekennzeichnet, dass** das Molekulargewicht des Polyols zwischen 95 und 350 liegt.

18. Kosmetische Zusammensetzung nach einem der vorhergehenden Ansprüche, **dadurch gekennzeichnet, dass** das Polyol bzw. die Polyole in der Zusammensetzung in einem Gehalt zwischen 0,1 und 30 Gew.-%, vorzugsweise zwischen 0,5 und 20 Gew.-% und noch weiter bevorzugt zwischen 1 und 15 Gew.-% des Gesamtgewichts der kosmetischen Zusammensetzung vorliegen kann bzw. können.

19. Kosmetische Zusammensetzung nach einem der vorhergehenden Ansprüche, **dadurch gekennzeichnet, dass** sie außerdem mindestens ein Silikon umfasst.

20. Kosmetische Zusammensetzung nach einem der vorhergehenden Ansprüche, **dadurch gekennzeichnet, dass** sie außerdem mindestens ein zusätzliches ionisches oder nichtionisches fixierendes Polymer umfasst.

21. Kosmetische Zusammensetzung nach einem der vorhergehenden Ansprüche, **dadurch gekennzeichnet, dass** sie eine Viskosität von mehr als 200 cps bei 25 °C bei einer Scherrate von 1 s⁻¹ aufweist.

22. Kosmetische Zusammensetzung nach Anspruch 21, **dadurch gekennzeichnet, dass** sie eine Viskosität zwischen 500 und 500.000 cps bei 25 °C, vorzugsweise zwischen 500 und 100.000 cps bei 25 °C und noch weiter bevorzugt zwischen 500 und 50.000 cps bei 25 °C bei einer Scherrate von 1 s⁻¹ aufweist.

23. Kosmetische Zusammensetzung nach einem der vorhergehenden Ansprüche, **dadurch gekennzeichnet, dass** sie außerdem mindestens einen kosmetischen Hilfsstoff umfasst, der aus anionischen oder nichtionischen Tensiden, Konditionierungsmitteln vom Ester-Typ, Antischaummitteln, feuchtigkeitsspendenden Mitteln, zartmachenden Mitteln, Weichmachern, silikonhaltigen oder nicht silikonhaltigen wasserlöslichen und fettlöslichen Sonnenschutzfiltern, permanenten oder temporären Farbstoffen, Duftstoffen, Peptisierungsmitteln, Konservierungsmitteln, Ceramiden und Pseudoceramiden, Vitaminen und Provitaminen, darunter Panthenol, Proteinen, Sequestrierungsmitteln, Lösungsvermittlern, Alkalinisierungsmitteln, Ansäuerungsmitteln, Korrosionsschutzmitteln, Nichtsilikon-Fettsubstanzen wie pflanzlichen, tierischen, mineralischen und synthetischen Ölen und Wachsen, Reduktionsmitteln oder Antioxidantien, Oxidationsmitteln, mineralischen Füllstoffen und Flitter ausgewählt ist.

24. Kosmetische Zusammensetzung nach einem der vorhergehenden Ansprüche, **dadurch gekennzeichnet, dass** es sich bei dem kosmetisch unbedenklichen Medium um ein wässriges, alkoholisches oder wässrigalkoholisches Medium handelt.

25. Verfahren zur kosmetischen Behandlung, **dadurch gekennzeichnet, dass** man eine Zusammensetzung nach einem der Ansprüche 1 bis 24 aufbringt, insbesondere auf das Haar.

26. Verfahren zur kosmetischen Behandlung nach Anspruch 25, **dadurch gekennzeichnet, dass** nach dem Aufbringen der Zusammensetzung nicht gespült wird.

27. Verwendung einer kosmetischen Zusammensetzung nach einem der Ansprüche 1 bis 24 zur Fixierung der Haare.

28. Verwendung einer kosmetischen Zusammensetzung nach einem der Ansprüche 1 bis 24 zur Pflege des Haars.

## Claims

1. Cosmetic composition for treating keratin fibres, in particular human keratin fibres such as the hair, comprising, in a cosmetically acceptable medium:
- at least one terpolymer comprising, among its monomers, at least one α,β-monoethylenically unsaturated carboxylic acid; at least one ester of an α,β-monoethylenically unsaturated carboxylic acid and at least one ester of an α,β-monoethylenically unsaturated carboxylic acid and of an oxyalkylenated fatty alcohol;
- at least one fixing polymer chosen from non-ionic fixing homopolymers, the ratio between the amounts, expressed as weight percentage relative to the total weight of the composition, of the terpolymer and the non-ionic fixing homopolymer being greater than or equal to 0.2,
- at least one polyol other than the non-ionic fixing homopolymer, and the ratio between the amounts, expressed as weight percentage relative to the total weight of the composition, of the terpolymer and the polyol being less than 1, and
- if the composition comprises in addition an anionic fixing polymer, then the terpolymer is in an amount sufficient to obtain a viscosity greater than 50 cps at 25°C at a shear rate of 1 s⁻¹.

2. Cosmetic composition according to Claim 1, **characterized in that** the terpolymer comprises, as monomer, at least one C₁-C₁₀ α,β-monoethylenically unsaturated carboxylic acid; at least one ester of a C₃-C₁₀ α,β-monoethylenically unsaturated carboxylic acid and of a C₁-C₄ alcohol and at least one ester of an α,β-monoethylenically unsaturated carboxylic acid and of an oxyalkylenated fatty alcohol which is C₁₀-C₂₆.

3. Cosmetic composition according to Claim 2, **characterized in that** the terpolymer is a methacrylic acid/ethyl acrylate/ oxyalkylenated stearyl methacrylate copolymer.

4. Cosmetic composition according to any one of Claims 1 to 3, **characterized in that** the molecular weight by weight of the terpolymers is between 500 and 20 000 000, preferably between 200 000 and 2 000 000 and even more preferentially between 400 000 and 800 000.

5. Cosmetic composition according to any one of Claims 1 to 4, **characterized in that** the terpolymer(s) is or are present in the cosmetic composition in an amount ranging from 0.05% to 30% by weight, preferably in an amount ranging from 0.1% to 15% by weight and even more preferentially in an amount ranging from 0.2% to 10% by weight, relative to the total weight of the composition.

6. Cosmetic composition according to any one of the preceding claims, **characterized in that** the non-ionic fixing homopolymer is chosen from:
- homopolymers of vinyl acetate or of vinyl alcohol;
- styrene homopolymers;
- polyalkyloxazolines;
- vinyllactam homopolymers;
- alkyl acrylate homopolymers and alkyl methacrylate homopolymers.

7. Cosmetic composition according to the preceding claim, **characterized in that** the molecular weight by number of the non-ionic fixing homopolymer is greater than 350.

8. Cosmetic composition according to any one of the preceding claims, **characterized in that** the non-ionic fixing homopolymer(s) is or are present in the composition in an amount ranging from 0.1% to 30%, preferably in an amount ranging from 0.2% to 20%, and even more preferentially in an amount ranging from 0.5% to 10% by weight, relative to the total weight of the composition.

9. Cosmetic composition according to any one of the preceding claims, **characterized in that** the polyols are of formula (II) : 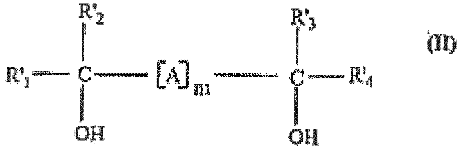 in which R'₁, R'₂, R'₃ and R'₄ denote, independently of one another, a hydrogen atom, a C₁-C₆ alkyl radical or a C₁-C₆ mono- or polyhydroxyalkyl radical,
A denotes a linear or branched alkylene radical containing from 1 to 18 carbon atoms, this radical comprising from 0 to 9 oxygen atoms but no hydroxyl group,
m denotes 0 or 1.

10. Cosmetic composition according to the preceding claim, **characterized in that**, when m is 0, then the polyol is chosen from 1,2,3-propanetriol, pinacol (2,3-dimethyl-2,3-butanediol), 1,2,3-butanetriol, 2,3-butanediol and sorbitol.

11. Cosmetic composition according to Claim 9, **characterized in that**, when m is 1, then R'₁, R'₂, R'₃ and R'₄ denote, independently of one another, a hydrogen atom or a C₁-C₆ alkyl radical.

12. Cosmetic composition according to the preceding claim, **characterized in that** the polyol is chosen from PEG-6 or PEG-8.

13. Cosmetic composition according to Claim 9, **characterized in that**, when m is 1, then R'₁, R'₂, R'₃ and R'₄ denote, independently of one another, a hydrogen atom or a C₁-C₆ alkyl radical, the molecular weight of the polyol being less than 200.

14. Cosmetic composition according to the preceding claim, **characterized in that** the polyol is chosen from 3-methyl-1,3,5-pentanetriol, 1,2,4-butanetriol, 1,5-pentanediol, 2-methyl-1,3-propanediol, 1,3-butanediol, 3-methyl-1,5-pentanediol, neopentyl glycol (2,2-dimethyl-1,3-propanediol), isoprene glycol (3-methyl-1,3-butanediol) and hexylene glycol (2-methyl-2,4-pentanediol) and even more preferably hexylene glycol, neopentyl glycol and 3-methyl-1,5-pentanediol.

15. Cosmetic composition according to any one of Claims 9 to 14, **characterized in that** the molecular weight of the polyol is between 75 and 350.

16. Cosmetic composition according to Claim 15, **characterized in that** the molecular weight of the polyol is between 90 and 350.

17. Cosmetic composition according to Claim 16, **characterized in that** the molecular weight of the polyol is between 95 and 350.

18. Cosmetic composition according to any one of the preceding claims, **characterized in that** the polyol(s) can be present in the composition at a content of between 0.1% and 30%, preferably between 0.5% and 20%, and even more preferentially between 1% and 15% by weight of the total weight of the cosmetic composition.

19. Cosmetic composition according to any one of the preceding claims, **characterized in that** it also comprises at least one silicone.

20. Cosmetic composition according to any one of the preceding claims, **characterized in that** it also comprises at least one ionic or non-ionic additional fixing polymer.

21. Cosmetic composition according to any one of the preceding claims, **characterized in that** it has a viscosity of greater than 200 cps at 25°C at a shear rate of 1 s⁻¹.

22. Cosmetic composition according to Claim 21, **characterized in that** it has a viscosity of between 500 and 500 000 cps at 25°C, preferably between 500 and 100 000 cps at 25°C, and even more preferentially between 500 and 50 000 cps at 25°C at a shear rate of 1 s⁻¹.

23. Cosmetic composition according to any one of the preceding claims, **characterized in that** it also comprises at least one cosmetic adjuvant chosen from anionic or non-ionic surfactants, ester-type conditioning agents, anti-foams, moisturizing agents, emollients, plasticizers, silicone or non-silicone, water-soluble and liposoluble sunscreens, permanent or temporary dyes, fragrances, peptizers, preservatives, ceramides and pseudoceramides, vitamins and provitamins, including panthenol, proteins, sequestrants, solubilizing agents, basifying agents, acidifying agents, anti-corrosion agents, non-silicone fatty substances such as vegetable, animal, mineral and synthetic oils, and waxes, reducing agents or antioxidants, oxidizing agents, mineral fillers and glitter flakes.

24. Cosmetic composition according to any one of the preceding claims, **characterized in that** the cosmetically acceptable medium is an aqueous, alcoholic or aqueous-alcoholic medium.

25. Cosmetic treatment process, **characterized in that** it comprises the application of a cosmetic composition according to any one of Claims 1 to 24, in particular to the hair.

26. Cosmetic treatment process according to Claim 25, **characterized in that** the application of said composition is not followed by rinsing.

27. Use of a cosmetic composition according to any one of Claims 1 to 24, for fixing the hair.

28. Use of a cosmetic composition according to any one of Claims 1 to 24, for haircare.
